# EUROPEAN PATENT APPLICATION

(11) **EP 3 943 512 A1**
(43) Date of publication of application: **26.01.2022**
(21) Application number: 20773078.9
(22) Date of filing: 18.03.2020
(51) Int. Cl.: C07K 19/00, C12N 15/11, A61K 48/00

(54) **FUSION PROTEIN FOR ENHANCING GENE EDITING AND USE THEREOF**

(30) Priority: 19.03.2019 CN 201910210107
(71) Applicant: Bioray Laboratories Inc, Shanghai 200241 (CN)
(72) Inventor: LI, Dali, Shanghai 200241 (CN); YIN, Shuming, Shanghai 200241 (CN); ZHANG, Mei, Shanghai 200241 (CN); CHEN, Xi, Shanghai 200241 (CN); ZHANG, Xiaohui, Shanghai 200241 (CN); WANG, Liren, Shanghai 200241 (CN); LIU, Mingyao, Shanghai 200241 (CN)
(74) Representative: Viering, Jentschura & Partner mbB Patent- und Rechtsanwälte
(86) International application number: PCT/CN2020/080032
(87) International publication number: WO 2020/187268

(57) **Abstract**

The present invention relates to a fusion protein for enhancing gene editing and use thereof. In particular, the invention provides an enhanced fusion protein. The enhanced fusion proteins of the present invention can significantly increase gene editing efficiency in vivo or in vitro as compared to the wildtype gene editing protein.

## Description

### Technical field

The present invention relates to the field of biotechnology, in particular to a fusion protein for enhancing gene editing and its application.

### Background

Gene editing is a genetic manipulation technology which modified genes by artificially achieving double-stranded DNA breaks and using the repair mechanism of double stranded DNA breaks. Current gene editing technologies include ZFN, TALEN and CRISPR/Cas9, among which CRISPR/Cas9 is the most widely applicability. CRISPR/Csa9 technology is a adaptive immune mechanism derived from bacteria or archaebacteria that uses a single-stranded guide RNA (sgRNA) and Cas9 protein to produce DNA double strand breaks at specific locations in the genome, then through endogenous non-homologous end binding (NHEJ) or homologous recombination (HDR) repair mechanism, the knockout of a target gene or the insertion of a specific gene or segment can be achieved.

Although the CRISPR/Cas9 technology is incredibly powerful, it also has some drawbacks, such as, 1. the concerns about off-target problem; 2. the limitation of PAM leads to the limitation of target site selection; 3. some newly developed tools ( XCas9 and SpCas9-NG) show generally low editing efficiency. However, the current optimization and modification of gene editing tools are mainly based on the improvement of the accuracy and targeting scope of the tools, and there are no methods to comprehensively improve various gene editing tools for the gene editing tools themselves.

Therefore, there is an urgent need in this field to develop a new method to improve the efficiency of gene editing.

### Summary of the invention

The purpose of the present invention is to provide a new method for improving gene editing efficiency.

In a first aspect of the present invention, it provides a fusion protein, the structure of the fusion protein is shown in the following Formula I or I':

C-A-L-B (I)

B-L-A-C (I')

wherein
A is a gene editing protein,
B is a DNA double-strand binding domain,
C is an optional base editor element;
L is none or a linker peptide;
each "-" is independently a linker peptide or a peptide bond or a non-peptide bond.

In another preferred embodiment, when the structure of the fusion protein is as shown in formula I', C is none.

In another preferred embodiment, the non-peptide bond includes PEG.

In another preferred embodiment, the gene editing protein is selected from the group consisting of Cas9, Cas12, Cas12a, Cas12b, Cas13, Cas14, and a combination thereof.

In another preferred embodiment, the gene editing protein includes wild type or mutant gene editing protein.

In another preferred embodiment, the gene editing protein is selected from the group consisting of *Streptococcus pyogenes, Staphylococcus aureus, Acidaminococcus sp, Lachnospiraceae bacterium,* and a combination thereof.

In another preferred embodiment, the amino acid sequence of the wild-type gene editing protein is shown in SEQ ID NO.: 1, 14 or 15.

In another preferred embodiment, the amino acid sequence of the base editor element is shown in SEQ ID NO.: 2 or 12.

In another preferred embodiment, the DNA double-strand binding domain is a non-sequence-specific DNA double-strand binding domain.

In another preferred embodiment, the DNA double-stranded binding domain is selected from the group consisting of HMG-D, Sac7d, and a combination thereof.

In another preferred embodiment, the DNA double-stranded binding domain includes a wild-type DNA double-stranded binding domain and a mutant DNA double-stranded binding domain.

In another preferred embodiment, the DNA double-stranded binding domain is derived from Drosophila or archaebacteria.

In another preferred embodiment, the amino acid sequence of the DNA double-strand binding domain is shown in SEQ ID NO.: 10 or 11.

In another preferred embodiment, the length of the linker peptide is 1-100 aa, preferably, 15-85 aa, more preferably, 25-70 aa.

In another preferred embodiment, the linker peptide is a sequence shown as Gly-Gly-Ser with n repeats, wherein n is 2-8, preferably n is 3-6.

In another preferred embodiment, the amino acid sequence of the linker peptide is selected from the group consisting of:
(1) a polypeptide having an amino acid sequence as shown in any one of SEQ ID NO.: 3-7; or
(2) a polypeptide formed by substituting, deleting or adding one or several, preferably 1-20, more preferably 1-15, more preferably 1-10, more preferably 1-8, more preferably 1-3, most preferably 1 amino acid residues to the amino acid sequence as shown in any one of SEQ ID NO.: 3-7, derived from a polypeptide having the amino acid sequence as shown in any one of SEQ ID NO.: 3-7 having the function of the polypeptide as described in (1).

In another preferred embodiment, the base editor element includes cytosine deaminase and adenine deaminase.

In another preferred embodiment, the cytosine deaminase includes Apobec1 and Apobec3A.

In another preferred embodiment, the adenine deaminase includes TadA.

In another preferred embodiment, the fusion protein has an amino acid sequence as shown in any one of SEQ ID NO.: 8, 9, and 13.

In a second aspect of the present invention, it provides a polynucleotide, which encodes the fusion protein according to the first aspect of the present invention.

In another preferred embodiment, the polynucleotide additionally contains an auxiliary element selected from the group consisting of signal peptide, secretory peptide, tag sequence (such as 6His), or a combination thereof on the flanking of the ORF of the mutant protein or fusion protein.

In another preferred embodiment, the polynucleotide is selected from the group consisting of: DNA sequence, RNA sequence, and a combination thereof.

In a third aspect of the present invention, it provides a vector containing the polynucleotide according to the second aspect of the present invention.

In another preferred embodiment, the vector includes one or more promoters, which are operably linked to the nucleic acid sequence, enhancer, transcription termination signal, polyadenylation sequence, origin of replication, selectable marker, nucleic acid restriction site, and/or homologous recombination site.

In another preferred embodiment, the vector includes a plasmid and viral vector.

In another preferred embodiment, the viral vector is selected from the group consisting of adeno-associated virus (AAV), adenovirus, lentivirus, retrovirus, herpes virus, SV40, poxvirus, and a combination thereof.

In another preferred embodiment, the vector includes an expression vector, a shuttle vector, and an integration vector.

In a fourth aspect of the present invention, it provides a host cell containing the vector according to the third aspect of the present invention, or its genome integrates the polynucleotide according to the second aspect of the present invention.

In another preferred embodiment, the host cell is a eukaryotic cell, such as a yeast cell, a plant cell or a mammalian cell (including a human and non-human mammal).

In another preferred embodiment, the host cell is a prokaryotic cell, such as Escherichia coli.

In another preferred embodiment, the yeast cell is selected from one or more yeasts from the group consisting of: Pichia pastoris, Kluyveromyces, and a combination thereof; preferably, the yeast cell includes: Kluyveromyces, more preferably Kluyveromyces marxianus, and/or Kluyveromyces lactis.

In another preferred embodiment, the host cell is selected from the group consisting of Escherichia coli, wheat germ cell, insect cell, SF9, Hela, HEK293, CHO, yeast cell, and a combination thereof.

In a fifth aspect of the present invention, it provides a method for producing the fusion protein according to the first aspect of the present invention, comprising the steps:
Under conditions suitable for expression, culturing the host cell according to the fourth aspect of the present invention, thereby expressing the fusion protein; and/or
Isolating the fusion protein.

In a sixth aspect of the present invention, it provides a gene editing reagent, which comprises the fusion protein according to the first aspect of the present invention.

In another preferred embodiment, the reagent further includes one or more reagents selected from the group consisting of:
(a1) gRNA, crRNA, or a vector for producing the gRNA or crRNA;
(a2) a template for homology-directed repair: single-stranded nucleotide sequence or plasmid vector.

In a seventh aspect of the present invention, it provides a kit comprising the gene editing reagent according to the sixth aspect of the present invention.

In another preferred embodiment, the kit further includes one or more reagents selected from group consisting of:
(a1) gRNA, crRNA, or a vector for producing the gRNA or crRNA;
(a2) a template for homology-directed repair: single-stranded nucleotide sequence or plasmid vector.

In another preferred embodiment, the kit further includes a label or instructions.

In an eighth aspect of the present invention, it provides a use of the fusion protein according to the first aspect of the present invention for the preparation of a reagent or kit for improving gene editing efficiency.

In a ninth aspect of the present invention, it provides a pharmaceutical composition, comprising:
(a) a fusion protein according to the first aspect of the present invention, or a coding gene thereof, or an expression vector (vector) thereof; and
(b) a pharmaceutically acceptable carrier (carrier).

In another preferred embodiment, the expression vector includes a viral vector.

In another preferred embodiment, the viral vector is selected from the group consisting of adeno-associated virus (AAV), adenovirus, lentivirus, retrovirus, herpes virus, SV40, poxvirus, and a combination thereof.

In another preferred embodiment, the vector is selected from the group consisting of: lentivirus, adenovirus, adeno-associated virus (AAV), and a combination thereof, preferably, the vector is adeno-associated virus (AAV).

In another preferred embodiment, the dosage form of the pharmaceutical composition is selected from the group consisting of a lyophilized preparation, a liquid preparation, and a combination thereof.

In another preferred embodiment, the dosage form of the pharmaceutical composition is an injection dosage form.

In another preferred embodiment, the pharmaceutical composition also includes other drugs for gene therapy.

In another preferred embodiment, the other drugs for gene therapy are selected from the group consisting of antisense nucleotide drugs, EDIT-101 drugs, CTX001, and a combination thereof.

In another preferred embodiment, the pharmaceutical composition is a cell preparation.

In a tenth aspect of the present invention, it provides a medicine kit comprising:
(a1) a first container, and the fusion protein according to the first aspect of the present invention, a coding gene thereof, or an expression vector thereof, or a medicine containing the fusion protein according to the first aspect of the present invention located in the first container .

In another preferred embodiment, the medicine kit further includes:
(a2) a second container, and other medicines for gene therapy, or medicines containing other medicines for gene therapy located in the second container.

In another preferred embodiment, the first container and the second container are the same or different containers.

In another preferred embodiment, the medicine located in the first container is a prescribed preparation containing the fusion protein of claim 1.

In another preferred embodiment, the medicine located in the second container is a prescribed preparation containing other medicines for gene therapy.

In another preferred embodiment, the dosage form of the medicine is selected from the group consisting of a lyophilized preparation, a liquid preparation, and a combination thereof.

In another preferred embodiment, the dosage form of the medicine is an injection dosage form.

In an eleventh aspect of the present invention, it provides a use of the fusion protein according to the first aspect of the present invention for the preparation of a medicine for gene therapy.

In a twelfth aspect of the present invention, it provides a method for improving gene editing efficiency, comprising the steps:
In the presence of the fusion protein according to the first aspect of the present invention or the gene editing reagent according to the sixth aspect of the present invention, performing gene editing on a cell, thereby improving the efficiency of gene editing.

In another preferred embodiment, the cell includes a human or non-human mammalian cell (such as a primate or livestock).

In another preferred embodiment, the cell includes a cancer cell or normal cell.

In another preferred embodiment, the cell is selected from the group consisting of a kidney cell, liver cell, nerve cell, heart cell, epithelial cell, muscle cell, somatic cell, bone marrow cell, endothelial cell, and a combination thereof.

In another preferred embodiment, the cell is selected from the group consisting of a 293 cell, A549 cell, SW626 cell, HT-3 cell, PA-1 cell, and a combination thereof.

In another preferred embodiment, the cell includes HEK293T.

In another preferred embodiment, the gene editing is performed in an in vitro reaction system.

In another preferred embodiment, in the in vitro reaction system, the content of the fusion protein or gene editing reagent is 100ng-700ng, preferably, 200ng-600ng, more preferably, 300ng-500ng.

In another preferred embodiment, the method is non-diagnostic and non-therapeutic.

In another preferred embodiment, the cell is an in vitro cell.

It should be understood that, within the scope of the present invention, the technical features specifically described above and below (such as the Examples) can be combined with each other, thereby constituting a new or preferred technical solution which needs not be described one by one.

### Description of Drawings

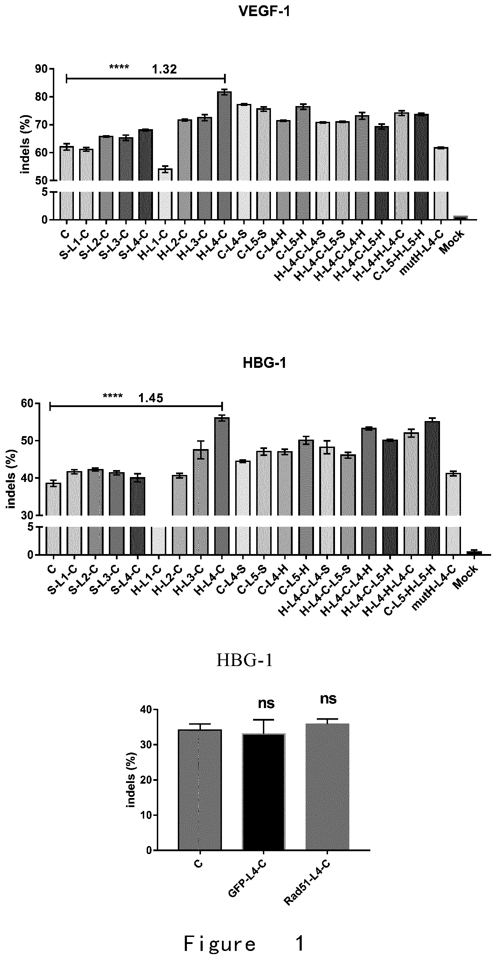

Figure 1 shows the editing efficiency of two different endogenous targets, and it shows that the different fusion architecture between the DNA double-stranded binding domain and Cas9 protein, as well as the various linker length, have a difference effects on efficiency improvement. Comprehensively, we choose the best fusion architecture that the HMG-D domain fused to the N-terminal of Cas9 via L4 length linker, i.e., HMG-D-L4-Cas9, wherein H represents HMG-D, S represents Sac7d, L1-L5 represents various length linker, mutH represents mutant HMG-D (V32A and T33A mutations reduce the binding activity), C represents Cas9.
Figure 2 shows HMG-D-L4-Cas9 can improve gene editing efficiency in the other endogenous target sites, and the efficiency improvement> 20%, preferably,> 40%, more preferably,> 60% (such as 80%), up to 2 times, wherein H represents HMG-D.
Figure 3 shows the double-strand binding domain HMG-D can improve the efficiency of Cas9 protein from other sources, like SaCas9, by more than 20%.
Figure 4 shows the double-strand binding domain HMG-D can improve the efficiency of non-Cas9 proteins (such as AsCas12a) by 10-20%..
Figure 5 shows the double-strand binding domain HMG-D can improve the efficiency of the epigenetic regulation tools (e.g., CRISPR-VPR), the efficiency can be increased by 2 times and wherein endCas9 is HMG-D-L4-dCas9.
Figure 6 shows the double-strand binding domain HMG-D can improve the efficiency of the single-base editing tool ABE, wherein H represents HMG-D.

### DETAILED DESCRIPTION

After extensive and in-depth research, the inventors have unexpectedly obtained an enhanced fusion protein. Compared with wild-type gene editing protein, the enhanced fusion protein of the present invention can significantly improve gene editing efficiency in vivo or in vitro, and the present invention has also unexpectedly discovered that fusion protein formed by gene editing protein and DNA double-strand binding domain, optional base editor element and optional linker peptide can significantly improve gene editing efficiency (increased by ≥20%, such as 80%, or even up to 2 times). In addition, the present invention has also unexpectedly discovered that the fusion protein of the present invention can be used in gene therapy. On this basis, the inventors have completed the present invention.

### Terms

In order to make it easier to understand the present disclosure, first defining certain terms. As used in this application, unless expressly stated otherwise herein, each of the following terms shall have the meaning given below. Other definitions are stated throughout the application.

The term "about" may refer to a value or composition within an acceptable error range of a particular value or composition determined by a person of ordinary skill in the art, which will depend in part on how the value or composition is measured or determined. For example, as used herein, the expression "about 100" includes all values between 99 and 101 (e.g., 99.1, 99.2, 99.3, 99.4, etc.).

As used herein, the term "containing" or "comprising (including)" can be open, semi-closed, and closed. In other words, the term also includes "substantially consisting of' or "consisting of'.

Sequence identity (or homology) is determined by comparing two aligned sequences along a predetermined comparison window (it can be 50%, 60%, 70%, 80%, 90%, 95% or 100% of the length of the reference nucleotide sequence or protein) and determining the number of positions where the same residue appears. Usually, this is expressed as a percentage. The measurement of sequence identity of nucleotide sequences is a method well known to those skilled in the art.

As used herein, the term "EDIT-101 drug" belongs to gene therapy drugs, which is a type of cell. Specifically, EDIT-101 is a drug that uses CRISPR gene editing technology to treat hereditary retinal degeneration disease (LCA10 disease), EDIT-101 is administered by subretinal injection, and the gene editing system is directly delivered to the photoreceptor cells to achieve the therapeutic effect.

As used herein, the term "CTX001" belongs to gene therapy drugs, which is a type of cell. Specifically, CTX001 is based on CRISPR gene editing technology to achieve therapeutic purposes by cutting the BCL11A gene of patients with β-thalassemia.

### Wild-type gene editing protein

As used herein, "wild-type gene editing protein" refers to a naturally occurring gene editing protein that has not been artificially modified. Its nucleotides can be obtained through genetic engineering techniques, such as genome sequencing, polymerase chain reaction (PCR ) etc.. The amino acid sequence can be deduced from the nucleotide sequence. The source of the wild-type gene editing protein includes (but is not limited to): *Streptococcus pyogenes, Staphylococcus aureus, Acidaminococcus sp, Lachnospiraceae bacterium* .

In a preferred embodiment of the present invention, the amino acid sequence of the wild-type gene editing protein is shown in SEQ ID NO.: 1 or 14 or 15.

In a preferred embodiment of the present invention, the gene editing protein includes, but is not limited to, Cas9, Cas9a, Cas12, Cas12a, Cas12b, Cas13, and Cas14.

### DNA double-strand binding domain

As used herein, the term "DNA double-strand binding domain" is a DNA double-strand binding domain without sequence specificity. Compared with the sequence-specific DNA double-stranded binding domain, the non-sequence-specific DNA double-stranded binding domain of the present invention is not limited by the DNA sequence, and theoretically can bind to any DNA sequence. Therefore, it can be applied to any position of DNA binding. A preferred sequence of DNA double-strand binding domain is shown in SEQ ID NO.: 10 or 11.

### Base editor

Any base editor provided herein can modify specific nucleotide bases without generating a significant proportion of insertion/deletion. As used herein, "insertion/deletion" refers to the insertion or deletion of nucleotide bases within a nucleic acid. Such insertions or deletions can lead to frameshift mutations in the coding region of the gene. In some embodiments, it is desirable to produce a base editor that effectively modifies (e.g., mutates or deamination) specific nucleotides within a nucleic acid without generating a large number of insertions or deletions (i.e., insertion/deletion) in the nucleic acid. In certain embodiments, any of the base editors provided herein are capable of producing a larger proportion of the intended modification (e.g., point mutations or deamination) relative to insertion/deletion.

Any base editor of the present invention can effectively generate intended mutations, such as point mutations, in nucleic acids (for example, nucleic acids in the genome) without generating a large number of unintended mutations, such as unintended point mutations.

In the present invention, the base editor includes cytosine deaminase and adenine deaminase, and other types of base editors as long as they have the functions of the base editor of the present invention are also within the protection scope of the present invention.

In the present invention, the structure after the gene editing protein is fused with the base editor is called ABE or CBE, wherein ABE is the structure of gene editing protein fused with adenine deaminase and CBE is the structure of gene editing protein fused with cytosine deaminase.

The sequence of a preferred base editor is shown in SEQ ID NO.: 2 or 12.

### Fusion protein

As used herein, "fusion protein of the present invention" or "polypeptide" refers to the fusion protein according to the second aspect of the present invention. The structure of the fusion protein of the present invention is shown in the following Formula I or I':

C-A-L-B (I)

B-L-A-C (I')

wherein
A is a gene editing protein,
B is a DNA double-strand binding domain,
C is an optional base editor element;
L is none or a linker peptide,
each "-" is independently a linker peptide or a peptide bond or a non-peptide bond.

In the present invention, the length of the linker peptide has an effect on the activity of the fusion protein. The preferred length of the linker peptide is 1-100 aa, preferably, 15-85 aa, and more preferably, 25-70 aa.

A preferred linker peptide is shown in SEQ ID NO.: 3-7.

As used herein, the term "fusion protein" also includes variant forms as shown in SEQ ID NO.: 8, 9, or 13 having the above-mentioned activity. These variant forms include (but are not limited to): 1-3 (usually 1-2, more preferably 1) amino acid deletions, insertions and/or substitutions, and adding or deleting one or several (usually within 3, preferably within 2, more preferably within 1) amino acid at the C-terminal and/or N-terminal. For example, in this field, when amino acids with close or similar properties are substituted, the function of the protein is usually not changed. For another example, adding or deleting one or several amino acids at the C-terminus and/or N-terminus usually does not change the structure and function of the protein. In addition, the term also includes the polypeptide of the present invention in monomeric and multimeric forms. The term also includes linear and non-linear polypeptides (such as cyclic peptides).

The present invention also includes active fragments, derivatives and analogs of the above-mentioned fusion protein. As used herein, the terms "fragment", "derivative" and "analog" refer to a polypeptide that substantially retains the function or activity of the fusion protein of the present invention. The polypeptide fragments, derivatives or analogues of the present invention can be (i) a polypeptide in which one or more conservative or non-conservative amino acid residues (preferably conservative amino acid residues) are substituted, or (ii) a polypeptide with a substitution group in one or more amino acid residues, or (iii) a polypeptide formed by fusion of an antigen peptide with another compound (such as a compound that prolongs the half-life of the polypeptide, such as polyethylene glycol), or (iv) the polypeptide formed by fusion of additional amino acid sequence to this polypeptide sequence (fusion protein formed by fusion with leader sequence, secretory sequence or 6His tag sequence). According to the teachings herein, these fragments, derivatives and analogs fall within the scope of those skilled in the art.

A preferred type of active derivative means that compared with the amino acid sequence of Formula I, there are at most 3, preferably at most 2, and more preferably at most 1 amino acid replaced by an amino acid with close or similar properties to form a polypeptide. These conservative variant polypeptides are best produced according to Table A by performing amino acid substitutions.

**Table A**

| Initial residues | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogs of the fusion protein of the present invention. The difference between these analogs and the polypeptide as shown in SEQ ID NO.: 8 or 9 or 13 may be a difference in amino acid sequence, may also be a difference in modified form that does not affect the sequence, or both. Analogs also include analogs having residues different from natural L-amino acids (such as D-amino acids), and analogs having non-naturally occurring or synthetic amino acids (such as β, γ-amino acids). It should be understood that the polypeptide of the present invention is not limited to the representative polypeptides exemplified above.

Modified (usually without changing the primary structure) forms include: chemically derived forms of polypeptides in vivo or in vitro, such as acetylation or carboxylation. Modifications also include glycosylation, such as those polypeptides produced by glycosylation modifications during the synthesis and processing of the polypeptide or during further processing steps. This modification can be accomplished by exposing the polypeptide to an enzyme that performs glycosylation (such as a mammalian glycosylase or deglycosylase). Modified forms also include sequences with phosphorylated amino acid residues (such as phosphotyrosine, phosphoserine, phosphothreonine). It also includes polypeptides that have been modified to improve their anti-proteolytic properties or optimize their solubility properties.

In the present invention, in Formula I, A is a gene editing protein, B is HMG-D or Sac7d, C is adenine deaminase or cytosine deaminase or none, and L is L1 or L2 or L3 or L4 or L5 or none.

In a preferred embodiment, in Formula I, A is a gene editing protein, B is HMG-D, C is either adenine deaminase or cytosine deaminase or none, and L is L4 or L5.

In a preferred embodiment, the fusion protein of the present invention may also include two or more of the A, B, C, and L elements in Formula I.

In a preferred embodiment, in Formula I, A is a gene editing protein, B is HMG-9, C is none, and L is L4.

In a preferred embodiment, in Formula I, A is a gene editing protein, B is HMG-D, C is adenine deaminase, and L is L5.

In a preferred embodiment, in Formula I, A is a gene editing protein, B is HMG-D, C is cytosine deaminase, and L is L5.

In a preferred embodiment, the amino acid sequence of the fusion protein of the present invention is as shown in SEQ ID NO.: 8, 9 or 13.

### Adeno-associated virus

Because Adeno-associated virus (AAV) is smaller than other viral vectors, and is non-pathogenic, and can be transfected into dividing and undivided cells, gene therapy methods for genetic diseases based on AAV vectors have received widespread attention.

Adeno-associated virus (AAV), also known as adeno associated virus, belongs to the family Parvoviridae and the genus Dependovirus. It is the simplest single-stranded DNA-deficient virus found so far, and requires helper viruses (usually adenoviruses) to participate in replication. It encodes cap and rep genes in the inverted repeat sequence (ITR) at both ends. ITRs play a decisive role in virus replication and packaging. Cap gene encodes virus capsid protein, and rep gene participates in virus replication and integration. AAV can infect a variety of cells.

Recombinant adeno-associated virus vector (rAAV) is derived from non-pathogenic wild-type adeno-associated virus. Due to its good safety, wide range of host cells (divided and non-divided cells), low immunogenicity, and long time to express foreign genes *in vivo,* it is regarded as one of the most promising gene transfer vectors and has been widely used in gene therapy and vaccine research worldwide. After more than 10 years of research, the biological characteristics of recombinant adeno-associated virus have been deeply understood, especially in terms of its application effects in various cells, tissues and *in vivo* experiments, in which a lot of information have been accumulated. In medical research, rAAV is used in gene therapy for various diseases (including *in vivo* and *in vitro* experiments). At the same time, as a characteristic gene transfer vector, it is also widely used in gene function research, disease model construction, gene knockout mouse preparation, etc.

In a preferred embodiment of the present invention, the vector is a recombinant AAV vector. AAVs are relatively small DNA viruses that can be integrated into the genome of the cells they infect in a stable and site-specific manner. They can infect a wide range of cells without any effect on cell growth, morphology or differentiation, and they do not seem to be involved in human pathology. AAV genome has been cloned, sequenced and characterized. AAV contains inverted terminal repeat (ITR) regions of about 145 bases at each end, which serve as the origin of replication of the virus. The rest of the genome is divided into two important regions with encapsidation functions: the left portion of the genome containing the rep gene involved in viral replication and viral gene expression; and the right portion of the genome comprising the cap gene encoding the viral capsid protein.

AAV vectors can be prepared using standard methods in the art. Any serotype of adeno-associated virus is suitable. Methods for purifying vectors can be found, for example, in U.S. Pat. Nos. 6,566,118, 6,989,264 and 6,995,006, the disclosures of which are incorporated herein by reference in their entirety. The preparation of hybrid vectors is described, for example, in PCT application No. PCT/US2005/027091, the disclosure of which is incorporated herein by reference in its entirety. The use of AAV-derived vectors for transporting genes *in vitro* and *in vivo* has been described (see, for example, International Patent Application Publication Nos. WO 91/18088 and WO 93/09239; U.S. Pat. Nos. 4,797,368, 6,596,535 and 5,139,941, and European Pat. No. 0488,528, which are incorporated herein by reference in their entirety). These patent publications describe various constructs derived from AAV in which the rep and/or cap genes are deleted and replaced by the gene of interest, and the use of these constructs to transport the gene of interest *in vitro* (into cultured cells) or *in vivo* (directly into organisms). Replication-deficient recombinant AAV can be prepared by co-transfecting the following plasmids into cell lines infected by human helper viruses (such as adenoviruses): plasmids containing nucleic acid sequences of interest flanked by two AAV inverted terminal repeat (ITR) regions, and plasmids carrying AAV capsizing genes (rep and cap genes). The resulting AAV recombinant was then purified by standard techniques.

In some embodiments, the recombinant vector is capsized into viral particles (for example, AAV virus particles including but not limited to AAV1, AAV2, AAV3, AAV4, AAV5, AAV6, AAV7, AAV8, AAV9, AAV10, AAV11, AAV12, AAV13, AAV14, AAV15 and AAV16). Accordingly, the present disclosure includes recombinant viral particles (recombinant because they contain recombinant polynucleotides) comprising any of the vectors described herein. Methods of producing such particles are known in the art and are described in U.S. Pat. No. 6,596,535.

### Expression vectors and host cells

The invention also relates to a vector containing the polynucleotide of the present invention and a host cell produced by genetic engineering using the vector of the present invention or the coding sequence of the fusion protein of the present invention, and a method for producing the polypeptide of the present invention by recombinant technology.

Through conventional recombinant DNA technology, the polynucleotide sequence of the present invention can be used to express or produce recombinant fusion protein. Generally speaking, there are the following steps:
(1) using the polynucleotide (or variant) of the present invention encoding the fusion protein of the present invention, or using a recombinant expression vector containing the polynucleotide to transform or transduce a suitable host cell;
(2). a host cell cultured in a suitable medium;
(3). separating and purifying protein from culture medium or cells.

In the present invention, the polynucleotide sequence encoding the fusion protein can be inserted into a recombinant expression vector. The term "recombinant expression vector" refers to bacterial plasmids, bacteriophages, yeast plasmids, plant cell viruses, mammalian cell viruses such as adenovirus, retrovirus or other vectors well known in the art. Any plasmid and vector can be used as long as it can be replicated and stabilized in the host. An important feature of an expression vector is that it usually contains an origin of replication, a promoter, a marker gene, and translation control elements.

Methods well known to those skilled in the art can be used to construct an expression vector containing the DNA sequence encoding the fusion protein of the present invention and appropriate transcription/translation control signals. These methods include in vitro recombinant DNA technology, DNA synthesis technology, and in vivo recombination technology. The DNA sequence can be effectively linked to an appropriate promoter in the expression vector to guide mRNA synthesis. Representative examples of these promoters are: Escherichia coli lac or trp promoter; lambda phage PL promoter; eukaryotic promoters including CMV immediate early promoter, HSV thymidine kinase promoter, early and late SV40 promoter, retroviral LTRs and some other known promoters that can control gene expression in prokaryotic or eukaryotic cells or viruses. The expression vector also includes a ribosome binding site for translation initiation and a transcription terminator.

In addition, the expression vector preferably contains one or more selectable marker genes to provide phenotypic traits for selecting transformed host cells, such as dihydrofolate reductase for eukaryotic cell culture, neomycin resistance, and green fluorescent protein (GFP), or tetracycline or ampicillin resistance for E. coli.

A vector containing the above-mentioned appropriate DNA sequence and an appropriate promoter or control sequence can be used to transform an appropriate host cell so that it can express the protein.

The host cell can be a prokaryotic cell, such as Escherichia coli; or a lower eukaryotic cell, or a higher eukaryotic cell, such as a yeast cell, a plant cell or a mammalian cell (including a human and non-human mammal). Representative examples include: Escherichia coli, wheat germ cell, insect cell, SF9, Hela, HEK293, CHO, yeast cells, etc.. In a preferred embodiment of the present invention, yeast cells (such as Pichia pastoris, Kluyveromyces, or a combination thereof; preferably, the yeast cells include: Kluyveromyces, more preferably Kluyveromyces marxianus, and/or Kluyveromyces lactis) are selected as host cells.

When the polynucleotide of the present invention is expressed in higher eukaryotic cells, if an enhancer sequence is inserted into the vector, the transcription will be enhanced. Enhancers are cis-acting factors of DNA, usually about 10 to 300 base pairs, acting on promoters to enhance gene transcription. Examples include the 100 to 270 base pair SV40 enhancer on the late side of the replication initiation point, the polyoma enhancer on the late side of the replication initiation point, and adenovirus enhancers and the like.

Those of ordinary skill in the art know how to select appropriate vectors, promoters, enhancers and host cells.

Transformation of host cells with recombinant DNA can be carried out by conventional techniques well known to those skilled in the art. When the host is a prokaryote such as Escherichia coli, competent cells that can absorb DNA can be harvested after the exponential growth phase and treated with the CaCl2 method. The steps used are well known in the art. Another method is to use MgCl2. If necessary, transformation can also be carried out by electroporation. When the host is a eukaryote, the following DNA transfection methods can be selected: calcium phosphate co-precipitation method, conventional mechanical methods such as microinjection, electroporation, liposome packaging, etc..

The obtained transformants can be cultured by conventional methods to express the polypeptide encoded by the gene of the present invention. Depending on the host cell used, the medium used in the culture can be selected from various conventional mediums. The culture is carried out under conditions suitable for the growth of the host cell. After the host cells have grown to an appropriate cell density, the selected promoter is induced by a suitable method (such as temperature conversion or chemical induction), and the cells are cultured for a period of time.

The recombinant polypeptide in the above method can be expressed in the cell or on the cell membrane, or secreted out of the cell. If necessary, using its physical, chemical and other characteristics to separate and purify the recombinant protein through various separation methods. These methods are well known to those skilled in the art. Examples of these methods include, but are not limited to: conventional renaturation treatment, treatment with protein precipitation agent (salting out method), centrifugation, bacteria broken through osmosis, ultra-treatment, ultra-centrifugation, molecular sieve chromatography (gel filtration), adsorption chromatography, ion exchange chromatography, high performance liquid chromatography (HPLC) and various other liquid chromatography techniques and combinations of these methods.

### Gene therapy

Gene Therapy for genetic diseases refers to the application of genetic engineering technology to introduce normal genes into patient cells to correct defective genes and cure the disease. The way of correction can be either to repair the defective gene in situ, or to transfer a functional normal gene into a certain part of the cell genome to replace the defective gene to play a role. Gene is the basic functional unit that carries biological genetic information, and is a specific sequence located on the chromosome. Certain technical methods or vectors must be used to introduce foreign genes into biological cells. The methods of gene transfer are divided into biological methods, physical methods and chemical methods. Adenovirus vectors are currently one of the most commonly used viral vectors for gene therapy. Gene therapy is mainly to treat those diseases that are serious threats to human health, including, but not limited to: genetic diseases (such as hemophilia, cystic fibrosis, family hypercholesterolemia, etc.), malignant tumors, cardiovascular diseases, infectious diseases (such as AIDS, rheumatoid, etc.). Gene therapy is a high-tech biomedical technology that introduces human normal genes or therapeutic genes into human target cells in a certain way to correct gene defects or exert therapeutic effects, so as to achieve the purpose of treating diseases. Gene therapy is different from conventional treatment methods: in general, the treatment of diseases is aimed at various symptoms caused by genetic abnormalities, while gene therapy is aimed at the causes of the disease--the abnormal gene itself. Target cells for gene therapy include, but are not limited to, somatic cells, bone marrow cells, liver cells, nerve cells, endothelial cells, and muscle cells.

In the present invention, the target gene is subjected to efficient gene editing (including gene insertion, replacement, etc.) through gene therapy, thereby restoring the normal expression of the gene or enhancing the expression of the gene, thereby treating related diseases.

### The main advantages of the present invention include:

(1) The present invention is first to demonstrate the fusion protein of the present invention can significantly improve gene editing efficiency in vivo or in vitro.
(2) The present invention is first to demonstrate the fusion protein of the present invention can significantly improve gene editing efficiency in vivo or in vitro. The increase rate is ≥20%, preferably, >40%, more preferably, >60% (such as 80%), up to 2 times.
(3) The present invention is first to demonstrate the in vitro transcription of mRNA of enhanced gene editing tool to improve the success of animal model construction.
(4) The present invention use AAV virus to package the enhanced gene editing tool, and express the protein of enhanced gene editing tools to improve the effect of disease treatment.
(5) The present invention is first to use the fusion of double-stranded DNA binding domain to improve the gene editing efficiency.
(6) The present invention is first to screen and find a double-stranded DNA binding domain (like HMG-D) that can efficiently improve gene editing efficiency, as well as its excellent fusion mode.
(7) This patent is first to discover that the double-stranded DNA binding domain can extensively improve gene editing efficiency of various gene editing tools.
(8) The enhanced gene editing tools in the present invention can improve the success rate of animal model construction, as well as gene therapy efficiency.
(9) The enhanced gene editing tools in the present invention also can apply to gene therapy.

The present invention will be further explained below in conjunction with specific embodiments. It should be understood that these embodiments are only used to illustrate the present invention and not to limit the scope of the present invention. The experimental methods without specific conditions in the following examples are usually based on conventional conditions, such as the conditions described in Sambrook et al., Molecular Cloning: Laboratory Manual (New York: Cold Spring Harbor Laboratory Press, 1989), or according to manufacturing The conditions suggested by the manufacturer. Unless otherwise specified, percentages and parts are weight percentages and parts by weight.

Unless otherwise specified, the reagents and materials in the examples of the present invention are all commercially available products.

General methods:
Method 1: (applicable to Example 1, Example 2 and Example 3.
   1. The density of HEK293T cells in a 24-well plate grows to 60-70%, and then an equimalor amount of plasmid was transfected into cells with transfection reagent PEI. The medium is changed for 8-10 hours, the cells were cultured for a period of time (Cas9 system cultured for 72 hours, base editing system cultured for 120 hours), harvesting the cells, and extracting the genome.
   2. Design suitable primers to amplify a sequence of 150-180 bp around the target site, subjected to Hitom library construction, deep sequencing, analysis and calculation of editing efficiency..
Method 2: (applicable to animal model construction in Example 4).
   1. In vitro transcription of the mRNA of the enhanced gene editing tool and the sgRNA of the corresponding target.
   2. mRNA and sgRNA were injected into mice embryos, then transferred into pseudopregnant mother mice to obtain F0 generation mice, identify genes, count the mutation rate of mouse genotypes, and calculate the success rate of model construction.
Method 3: (applicable to gene therapy in Example 5).
   1. Packaging adeno-associated virus (AAV) of enhanced gene editing tools and the sgRNA of the therapeutic gene, or the homologous repaired template.
   2. Through intravenous injection (tail vein) or local injection (muscle, etc.) packaged AAV to the disease animal model (mouse or rat), and the control virus was also injected at the same time.
   3. Regularly observation and test of the phenotype of the animal models in treatment group and control group to evaluate the efficiency of treatment.

### Example 1: screening enhanced gene editing tools

To comparing the two endogenous targets (VEGF and HBG1/2), we synthesized various double-stranded DNA binding domain (HMG-D and Sac7d) respectively and designed five different lengths of linker (LI, L2, L3, L4 and L5) (Table 1), as well as fused to the N-terminus or C-terminus of Cas9. According to the resulting statistics, we scored the enhanced gene editing tools obtained from this series of optimizations (relative score based on the good results) (Bad: A; Not Good: AA; Better: AAA; Good: AAAA; Very Good: AAAAA), and the results are as follows: (note: C represents Cas9; L1-L5 represent various length linker; H represents HMG-D domain; S represents Sac7d)
A: S-L1-C, H-L1-C
AA: S-L2-C, S-L3-C, S-L4-C, H-L2-C
AAA: H-L4-C-L4-S, H-L4-C-L5-S
AAAA: H-L3-C, C-L4-S, C-L5-S, C-L4-H, H-L4-C-L4-H, H-L4-C-L5-H, H-L4-H-L4-C, C-L5-H-L5-H
AAAAA: H-L4-C, C-L5-H

After comprehensive statistics, we have found HMG-D works very well, and the the linker of L4 and L5 length (32 and 64 amino acids) is the most optimal linker. Through comparing the N-terminal and C-terminal connections, we have found the effects of the N-terminal and C-terminal connections are both very good under the optimal linker condition. Therefore, the inventors have developed an enhanced gene editing tools, HMG-D is fused to the N-terminal or C-terminal of Cas9 through the L4 or L5 linker, which is a very good fusion method (Figure 1).

Similarly, this enhanced gene editing tool, which enhances the efficiency of gene editing by fusing the double-stranded DNA binding domain, can also be applied to other types of double-stranded DNA binding domains, such as widely used zinc finger protein (ZFP), DNA binding domains of other transcription factors, and HMG-D or Sac7d from other species, etc.. The present invention can also improve the efficiency of gene editing through these double-stranded DNA binding domains. Therefore, the most important thing of the present invention is that the double-stranded DNA binding domain fused gene editing tool can improve the efficiency of gene editing, and wherein the double-stranded DNA binding domain HMG-D is preferred.

### Example 2: improving the gene editing efficiency of SpCas9

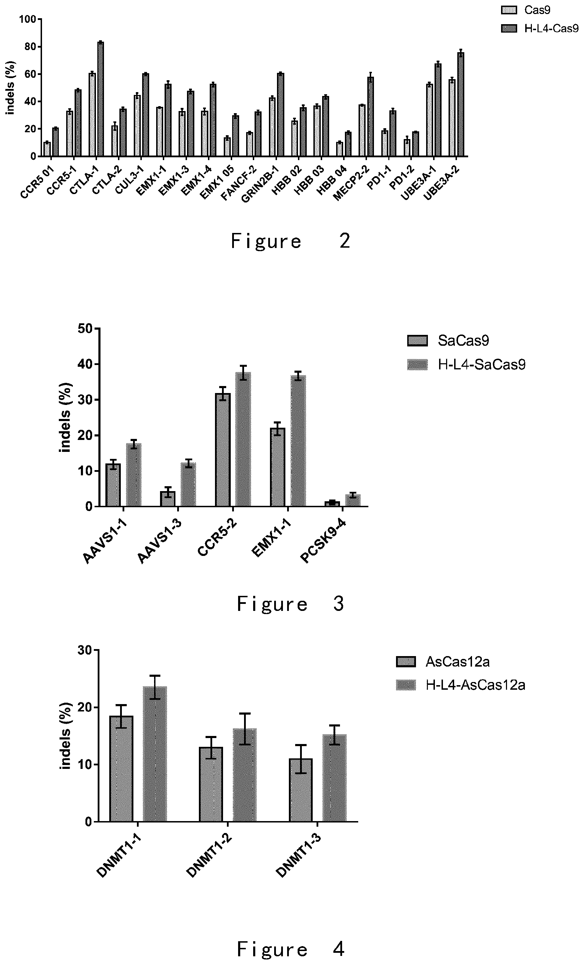

The obtained enhanced gene editing tool (i.e., the fusion protein of the present invention, such as HMG-D-L4-SpCas9) is further compared in terms of effect on more endogenous targets. By transfecting 293T cells with an equimolar ratio, it is found that compared with SpCas9, the editing efficiency of the fusion protein of the present invention has been improved by more than 20% (or 60%, or 80%), up to 2 times (Figure 2) in the compared targets..

### Example 3: improving the gene editing efficiency of Cas9 protein originated other species (like SaCas9)

Trough constructing HMG-D-L4-SaCas9 expression vector and transfecting 293T cells at an equimolar ratio to target the endogenous target, we have found the editing efficiency of SaCas9 after HMG-D fusion also improves similarly (Figure 3).

### Example 4: improving the gene editing efficiency of the other non-Cas9 protein (like AsCas12a)

Trough constructing HMG-D-L4-AsCas12a expression vector and transfecting 293T cells at an equimolar ratio to target the endogenous target, we have found the editing efficiency of AsCas12a after HMG-D fusion also improves similarly (Figure 4).

### Example 5: improving the transcriptional activation efficiency of the transcriptional regulatory tools (CRISPR/Cas9)

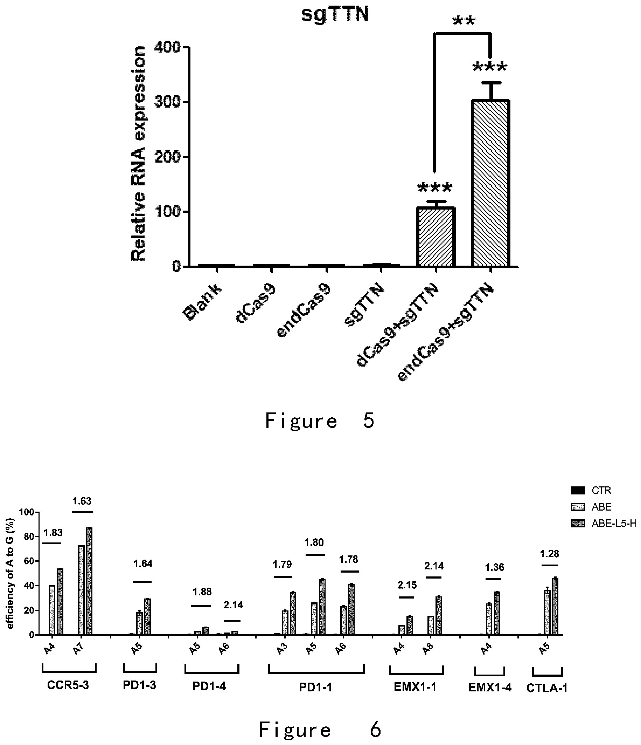

Trough constructing HMG-D-L4-dCas9-VPR expression vector and transfecting 293T cells at an equimolar ratio to target the endogenous target, we have found dCas9-VPR has a similar improvement effect on the transcriptional activation efficiency of endogenous genes after fusion of HMG-D. (Figure 5).

### Example 6: improving the editing efficiency of base editors

Since the base editor were developed by fusing cytosine deaminase (CBE) or adenine deaminase (ABE) to the N-terminal of Cas9, Therefore, at the C-terminal of Cas9 of the base editor, HMG-D was fused with the L5 linker to develop an enhanced single-base editing tool. Through the comparison of endogenous targets, we have found the editing efficiency of base editor (such as ABE) fused HMG-D have a huge improvement, such as by more than 1.5-2 times (Figure 6). The improvement in editing efficiency of fusion proteins fused with other types of base editors is similar to or comparable to that of fusion proteins fused with ABE.

### Example 7: improving the success rate of animal model construction, and the efficiency of gene therapy

Through in vitro transcription of mRNA of the enhanced gene editing tools, improving the success rate of animal model construction. Meanwhile, through packaging AAV virus of enhanced gene editing tools, the efficiency of disease treatment can also be improved.

### Comparative example 1: fusion of non-DNA binding domain show poor effect

To further validate the fusion of DNA binding domain can improve gene editing efficiency, we replaced DNA binding domain with the GFP protein (a non-DNA binding domain), an unrelated protein, and has found that the fusion of GFP can not improve gene editing efficiency (Figure 1). Meanwhile, the HMG-D domain was mutated to destroy its DNA binding ability, and a 3 amino acid mutant HMG-D domain (mutHMG-D, referred to as mutH) was constructed. Through experimental comparison, the mutHMG-D domain can not improve the efficiency of gene editing (Figure 1).

### Comparative example 2: apart from HMG-D and Sac7d, other DNA binding domain have poor effect

To expand the scope of DNA binding domain, we also tested some single-stranded DNA binding domain (such as Rad51), and has found the fusion of Rad51 can not improve gene editing efficiency (Figure 1). Hence, above results show the double-stranded DNA binding domain of HMG-D and Sac7d screened in the present invention are very effective.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. A fusion protein, wherein the structure of the fusion protein is shown in the following Formula I or I':
C-A-L-B (I)
B-L-A-C (I')
wherein A is a gene editing protein,
B is a DNA double-strand binding domain,
C is an optional base editor element;
L is none or a linker peptide;
each "-" is independently a linker peptide or a peptide bond or a non-peptide bond.

2. A polynucleotide, which encodes the fusion protein of claim 1.

3. A vector containing the polynucleotide of claim 2.

4. A host cell containing the vector of claim 3, or its genome integrates the polynucleotide of claim 2.

5. A method for producing the fusion protein of claim 1, comprising the steps:
Under conditions suitable for expression, culturing the host cell of claim 4, thereby expressing the fusion protein; and/or
Isolating the fusion protein.

6. A gene editing reagent, which comprises the fusion protein of claim 1.

7. A kit comprising the gene editing reagent of claim 6.

8. Use of the fusion protein of claim 1 for the preparation of a reagent or kit for improving gene editing efficiency.

9. a pharmaceutical composition, comprising:
(a) a fusion protein of claim 1, or a coding gene thereof, or an expression vector (vector) thereof; and
(b) a pharmaceutically acceptable carrier.

10. a medicine kit comprising:
(a1) a first container, and the fusion protein of claim 1, a coding gene thereof, or an expression vector thereof, or a medicine containing the fusion protein of claim 1 located in the first container .
In another preferred embodiment, the medicine kit further includes:
(a2) a second container, and other medicines for gene therapy, or medicines containing other medicines for gene therapy located in the second container.

11. Use of the fusion protein of claim 1 for the preparation of a medicine for gene therapy.

12. A method for improving gene editing efficiency, comprising the steps:
In the presence of the fusion protein of claim 1 or the gene editing reagent of claim 6, performing gene editing on a cell, thereby improving the efficiency of gene editing.


sequence listing

<110>  BIORAY LABORATORIES INC.
<120>  FUSION PROTEIN FOR ENHANCING GENE EDITING AND USE THEREOF
<1[?]0>  [?][?]2[?]0[?][?]3[?]0[?]8.9
<15[?]>  [?]N[?]9[?]9[?]0[?][?]0107.1

<160>  15

<170>  PatentIn version 3.5

<210>  1
<211>  1423
<212>  PRT
<213>  Streptococcus pyogenes

<400>  1

Met Asp Tyr Lys Asp His Asp Gly Asp Tyr Lys Asp His Asp Ile Asp
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Asp Lys Lys Tyr Ser Ile Gly Leu
        35                  40                  45

Asp Ile Gly Thr Asn Ser Val Gly Trp Ala Val Ile Thr Asp Glu Tyr
    50                  55                  60

Lys Val Pro Ser Lys Lys Phe Lys Val Leu Gly Asn Thr Asp Arg His
65                  70                  75                  80

Ser Ile Lys Lys Asn Leu Ile Gly Ala Leu Leu Phe Asp Ser Gly Glu
                85                  90                  95

Thr Ala Glu Ala Thr Arg Leu Lys Arg Thr Ala Arg Arg Arg Tyr Thr
            100                 105                 110

Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu

180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415

Pro Ile Leu Glu Lys Met Asp Gly Thr Glu Glu Leu Leu Val Lys Leu
            420                 425                 430

Asn Arg Glu Asp Leu Leu Arg Lys Gln Arg Thr Phe Asp Asn Gly Ser
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480

Lys Ile Leu Thr Phe Arg Ile Pro Tyr Tyr Val Gly Pro Leu Ala Arg
                485                 490                 495

Gly Asn Ser Arg Phe Ala Trp Met Thr Arg Lys Ser Glu Glu Thr Ile
            500                 505                 510

Thr Pro Trp Asn Phe Glu Glu Val Val Asp Lys Gly Ala Ser Ala Gln
        515                 520                 525

Ser Phe Ile Glu Arg Met Thr Asn Phe Asp Lys Asn Leu Pro Asn Glu
    530                 535                 540

Lys Val Leu Pro Lys His Ser Leu Leu Tyr Glu Tyr Phe Thr Val Tyr
545                 550                 555                 560

Asn Glu Leu Thr Lys Val Lys Tyr Val Thr Glu Gly Met Arg Lys Pro
                565                 570                 575

Ala Phe Leu Ser Gly Glu Gln Lys Lys Ala Ile Val Asp Leu Leu Phe
            580                 585                 590

Lys Thr Asn Arg Lys Val Thr Val Lys Gln Leu Lys Glu Asp Tyr Phe
        595                 600                 605

Lys Lys Ile Glu Cys Phe Asp Ser Val Glu Ile Ser Gly Val Glu Asp
    610                 615                 620

Arg Phe Asn Ala Ser Leu Gly Thr Tyr His Asp Leu Leu Lys Ile Ile
625                 630                 635                 640

Lys Asp Lys Asp Phe Leu Asp Asn Glu Glu Asn Glu Asp Ile Leu Glu
                645                 650                 655

Asp Ile Val Leu Thr Leu Thr Leu Phe Glu Asp Arg Glu Met Ile Glu
            660                 665                 670

Glu Arg Leu Lys Thr Tyr Ala His Leu Phe Asp Asp Lys Val Met Lys
        675                 680                 685

Gln Leu Lys Arg Arg Arg Tyr Thr Gly Trp Gly Arg Leu Ser Arg Lys
    690                 695                 700

Leu Ile Asn Gly Ile Arg Asp Lys Gln Ser Gly Lys Thr Ile Leu Asp
705                 710                 715                 720

Phe Leu Lys Ser Asp Gly Phe Ala Asn Arg Asn Phe Met Gln Leu Ile
                725                 730                 735

His Asp Asp Ser Leu Thr Phe Lys Glu Asp Ile Gln Lys Ala Gln Val
            740                 745                 750

Ser Gly Gln Gly Asp Ser Leu His Glu His Ile Ala Asn Leu Ala Gly
        755                 760                 765

Ser Pro Ala Ile Lys Lys Gly Ile Leu Gln Thr Val Lys Val Val Asp
    770                 775                 780

Glu Leu Val Lys Val Met Gly Arg His Lys Pro Glu Asn Ile Val Ile
785                 790                 795                 800

Glu Met Ala Arg Glu Asn Gln Thr Thr Gln Lys Gly Gln Lys Asn Ser
                805                 810                 815

Arg Glu Arg Met Lys Arg Ile Glu Glu Gly Ile Lys Glu Leu Gly Ser
            820                 825                 830

Gln Ile Leu Lys Glu His Pro Val Glu Asn Thr Gln Leu Gln Asn Glu
        835                 840                 845

Lys Leu Tyr Leu Tyr Tyr Leu Gln Asn Gly Arg Asp Met Tyr Val Asp
    850                 855                 860

Gln Glu Leu Asp Ile Asn Arg Leu Ser Asp Tyr Asp Val Asp His Ile
865                 870                 875                 880

Val Pro Gln Ser Phe Leu Lys Asp Asp Ser Ile Asp Asn Lys Val Leu
                885                 890                 895

Thr Arg Ser Asp Lys Asn Arg Gly Lys Ser Asp Asn Val Pro Ser Glu
            900                 905                 910

Glu Val Val Lys Lys Met Lys Asn Tyr Trp Arg Gln Leu Leu Asn Ala
        915                 920                 925

Lys Leu Ile Thr Gln Arg Lys Phe Asp Asn Leu Thr Lys Ala Glu Arg
    930                 935                 940

Gly Gly Leu Ser Glu Leu Asp Lys Ala Gly Phe Ile Lys Arg Gln Leu
945                 950                 955                 960

Val Glu Thr Arg Gln Ile Thr Lys His Val Ala Gln Ile Leu Asp Ser
                965                 970                 975

Arg Met Asn Thr Lys Tyr Asp Glu Asn Asp Lys Leu Ile Arg Glu Val
            980                 985                 990

Lys Val Ile Thr Leu Lys Ser Lys  Leu Val Ser Asp Phe  Arg Lys Asp
        995                 1000                1005

Phe Gln  Phe Tyr Lys Val Arg  Glu Ile Asn Asn Tyr  His His Ala
    1010                 1015                 1020

His Asp  Ala Tyr Leu Asn Ala  Val Val Gly Thr Ala  Leu Ile Lys
    1025                 1030                 1035

Lys Tyr  Pro Lys Leu Glu Ser  Glu Phe Val Tyr Gly  Asp Tyr Lys
    1040                 1045                 1050

Val Tyr  Asp Val Arg Lys Met  Ile Ala Lys Ser Glu  Gln Glu Ile
    1055                 1060                 1065

Gly Lys  Ala Thr Ala Lys Tyr  Phe Phe Tyr Ser Asn  Ile Met Asn
    1070                 1075                 1080

Phe Phe  Lys Thr Glu Ile Thr  Leu Ala Asn Gly Glu  Ile Arg Lys
    1085                 1090                 1095

Arg Pro  Leu Ile Glu Thr Asn  Gly Glu Thr Gly Glu  Ile Val Trp
    1100                 1105                 1110

Asp Lys  Gly Arg Asp Phe Ala  Thr Val Arg Lys Val  Leu Ser Met
    1115                 1120                 1125

Pro Gln  Val Asn Ile Val Lys  Lys Thr Glu Val Gln  Thr Gly Gly
    1130                 1135                 1140

Phe Ser  Lys Glu Ser Ile Leu  Pro Lys Arg Asn Ser  Asp Lys Leu
    1145                 1150                 1155

Ile Ala  Arg Lys Lys Asp Trp  Asp Pro Lys Lys Tyr  Gly Gly Phe
    1160                 1165                 1170

Asp Ser  Pro Thr Val Ala Tyr  Ser Val Leu Val Val  Ala Lys Val

1175                1180                1185

Glu Lys Gly Lys Ser Lys Lys Leu Lys Ser Val Lys Glu Leu Leu
    1190                1195                1200

Gly Ile Thr Ile Met Glu Arg Ser Ser Phe Glu Lys Asn Pro Ile
    1205                1210                1215

Asp Phe Leu Glu Ala Lys Gly Tyr Lys Glu Val Lys Lys Asp Leu
    1220                1225                1230

Ile Ile Lys Leu Pro Lys Tyr Ser Leu Phe Glu Leu Glu Asn Gly
    1235                1240                1245

Arg Lys Arg Met Leu Ala Ser Ala Gly Glu Leu Gln Lys Gly Asn
    1250                1255                1260

Glu Leu Ala Leu Pro Ser Lys Tyr Val Asn Phe Leu Tyr Leu Ala
    1265                1270                1275

Ser His Tyr Glu Lys Leu Lys Gly Ser Pro Glu Asp Asn Glu Gln
    1280                1285                1290

Lys Gln Leu Phe Val Glu Gln His Lys His Tyr Leu Asp Glu Ile
    1295                1300                1305

Ile Glu Gln Ile Ser Glu Phe Ser Lys Arg Val Ile Leu Ala Asp
    1310                1315                1320

Ala Asn Leu Asp Lys Val Leu Ser Ala Tyr Asn Lys His Arg Asp
    1325                1330                1335

Lys Pro Ile Arg Glu Gln Ala Glu Asn Ile Ile His Leu Phe Thr
    1340                1345                1350

Leu Thr Asn Leu Gly Ala Pro Ala Ala Phe Lys Tyr Phe Asp Thr
    1355                1360                1365

Thr Ile Asp Arg Lys Arg Tyr Thr Ser Thr Lys Glu Val Leu Asp
    1370                1375                1380

Ala Thr Leu Ile His Gln Ser Ile Thr Gly Leu Tyr Glu Thr Arg
    1385                1390                1395

Ile Asp Leu Ser Gln Leu Gly Gly Asp Lys Arg Pro Ala Ala Thr
    1400                1405                1410

Lys Lys  Ala Gly Gln Ala Lys  Lys Lys Lys
    1415                1420

<210>  2
<211>  1792
<212>  PRT
<213>  artificial sequence

<220>
<223>   base editor element

<400>  2

Met Pro Lys Lys Lys Arg Lys Val Ser Glu Val Glu Phe Ser His Glu
1               5                   10                  15

Tyr Trp Met Arg His Ala Leu Thr Leu Ala Lys Arg Ala Trp Asp Glu
            20                  25                  30

Arg Glu Val Pro Val Gly Ala Val Leu Val His Asn Asn Arg Val Ile
        35                  40                  45

Gly Glu Gly Trp Asn Arg Pro Ile Gly Arg His Asp Pro Thr Ala His
    50                  55                  60

Ala Glu Ile Met Ala Leu Arg Gln Gly Gly Leu Val Met Gln Asn Tyr
65                  70                  75                  80

Arg Leu Ile Asp Ala Thr Leu Tyr Val Thr Leu Glu Pro Cys Val Met
                85                  90                  95

Cys Ala Gly Ala Met Ile His Ser Arg Ile Gly Arg Val Val Phe Gly
            100                 105                 110

Ala Arg Asp Ala Lys Thr Gly Ala Ala Gly Ser Leu Met Asp Val Leu
        115                 120                 125

His His Pro Gly Met Asn His Arg Val Glu Ile Thr Glu Gly Ile Leu
    130                 135                 140

Ala Asp Glu Cys Ala Ala Leu Leu Ser Asp Phe Phe Arg Met Arg Arg
145                 150                 155                 160

Gln Glu Ile Lys Ala Gln Lys Lys Ala Gln Ser Ser Thr Asp Ser Gly
                165                 170                 175

Gly Ser Ser Gly Gly Ser Ser Gly Ser Glu Thr Pro Gly Thr Ser Glu
            180                 185                 190

Ser Ala Thr Pro Glu Ser Ser Gly Gly Ser Ser Gly Gly Ser Ser Glu

        195                 200                 205

Val Glu Phe Ser His Glu Tyr Trp Met Arg His Ala Leu Thr Leu Ala
    210                 215                 220

Lys Arg Ala Arg Asp Glu Arg Glu Val Pro Val Gly Ala Val Leu Val
225                 230                 235                 240

Leu Asn Asn Arg Val Ile Gly Glu Gly Trp Asn Arg Ala Ile Gly Leu
                245                 250                 255

His Asp Pro Thr Ala His Ala Glu Ile Met Ala Leu Arg Gln Gly Gly
            260                 265                 270

Leu Val Met Gln Asn Tyr Arg Leu Ile Asp Ala Thr Leu Tyr Val Thr
        275                 280                 285

Phe Glu Pro Cys Val Met Cys Ala Gly Ala Met Ile His Ser Arg Ile
    290                 295                 300

Gly Arg Val Val Phe Gly Val Arg Asn Ala Lys Thr Gly Ala Ala Gly
305                 310                 315                 320

Ser Leu Met Asp Val Leu His Tyr Pro Gly Met Asn His Arg Val Glu
                325                 330                 335

Ile Thr Glu Gly Ile Leu Ala Asp Glu Cys Ala Ala Leu Leu Cys Tyr
            340                 345                 350

Phe Phe Arg Met Pro Arg Gln Val Phe Asn Ala Gln Lys Lys Ala Gln
        355                 360                 365

Ser Ser Thr Asp Ser Gly Gly Ser Ser Gly Gly Ser Ser Gly Ser Glu
    370                 375                 380

Thr Pro Gly Thr Ser Glu Ser Ala Thr Pro Glu Ser Ser Gly Gly Ser
385                 390                 395                 400

Ser Gly Gly Ser Asp Lys Lys Tyr Ser Ile Gly Leu Ala Ile Gly Thr
                405                 410                 415

Asn Ser Val Gly Trp Ala Val Ile Thr Asp Glu Tyr Lys Val Pro Ser
            420                 425                 430

Lys Lys Phe Lys Val Leu Gly Asn Thr Asp Arg His Ser Ile Lys Lys
        435                 440                 445

Asn Leu Ile Gly Ala Leu Leu Phe Asp Ser Gly Glu Thr Ala Glu Ala
    450                 455                 460

Thr Arg Leu Lys Arg Thr Ala Arg Arg Arg Tyr Thr Arg Arg Lys Asn
465                 470                 475                 480

Arg Ile Cys Tyr Leu Gln Glu Ile Phe Ser Asn Glu Met Ala Lys Val
                485                 490                 495

Asp Asp Ser Phe Phe His Arg Leu Glu Glu Ser Phe Leu Val Glu Glu
            500                 505                 510

Asp Lys Lys His Glu Arg His Pro Ile Phe Gly Asn Ile Val Asp Glu
        515                 520                 525

Val Ala Tyr His Glu Lys Tyr Pro Thr Ile Tyr His Leu Arg Lys Lys
    530                 535                 540

Leu Val Asp Ser Thr Asp Lys Ala Asp Leu Arg Leu Ile Tyr Leu Ala
545                 550                 555                 560

Leu Ala His Met Ile Lys Phe Arg Gly His Phe Leu Ile Glu Gly Asp
                565                 570                 575

Leu Asn Pro Asp Asn Ser Asp Val Asp Lys Leu Phe Ile Gln Leu Val
            580                 585                 590

Gln Thr Tyr Asn Gln Leu Phe Glu Glu Asn Pro Ile Asn Ala Ser Gly
        595                 600                 605

Val Asp Ala Lys Ala Ile Leu Ser Ala Arg Leu Ser Lys Ser Arg Arg
    610                 615                 620

Leu Glu Asn Leu Ile Ala Gln Leu Pro Gly Glu Lys Lys Asn Gly Leu
625                 630                 635                 640

Phe Gly Asn Leu Ile Ala Leu Ser Leu Gly Leu Thr Pro Asn Phe Lys
                645                 650                 655

Ser Asn Phe Asp Leu Ala Glu Asp Ala Lys Leu Gln Leu Ser Lys Asp
            660                 665                 670

Thr Tyr Asp Asp Asp Leu Asp Asn Leu Leu Ala Gln Ile Gly Asp Gln
        675                 680                 685

Tyr Ala Asp Leu Phe Leu Ala Ala Lys Asn Leu Ser Asp Ala Ile Leu
    690                 695                 700

Leu Ser Asp Ile Leu Arg Val Asn Thr Glu Ile Thr Lys Ala Pro Leu
705                 710                 715                 720

Ser Ala Ser Met Ile Lys Arg Tyr Asp Glu His His Gln Asp Leu Thr
                725                 730                 735

Leu Leu Lys Ala Leu Val Arg Gln Gln Leu Pro Glu Lys Tyr Lys Glu
            740                 745                 750

Ile Phe Phe Asp Gln Ser Lys Asn Gly Tyr Ala Gly Tyr Ile Asp Gly
        755                 760                 765

Gly Ala Ser Gln Glu Glu Phe Tyr Lys Phe Ile Lys Pro Ile Leu Glu
    770                 775                 780

Lys Met Asp Gly Thr Glu Glu Leu Leu Val Lys Leu Asn Arg Glu Asp
785                 790                 795                 800

Leu Leu Arg Lys Gln Arg Thr Phe Asp Asn Gly Ser Ile Pro His Gln
                805                 810                 815

Ile His Leu Gly Glu Leu His Ala Ile Leu Arg Arg Gln Glu Asp Phe
            820                 825                 830

Tyr Pro Phe Leu Lys Asp Asn Arg Glu Lys Ile Glu Lys Ile Leu Thr
        835                 840                 845

Phe Arg Ile Pro Tyr Tyr Val Gly Pro Leu Ala Arg Gly Asn Ser Arg
    850                 855                 860

Phe Ala Trp Met Thr Arg Lys Ser Glu Glu Thr Ile Thr Pro Trp Asn
865                 870                 875                 880

Phe Glu Glu Val Val Asp Lys Gly Ala Ser Ala Gln Ser Phe Ile Glu
                885                 890                 895

Arg Met Thr Asn Phe Asp Lys Asn Leu Pro Asn Glu Lys Val Leu Pro
            900                 905                 910

Lys His Ser Leu Leu Tyr Glu Tyr Phe Thr Val Tyr Asn Glu Leu Thr
        915                 920                 925

Lys Val Lys Tyr Val Thr Glu Gly Met Arg Lys Pro Ala Phe Leu Ser
    930                 935                 940

Gly Glu Gln Lys Lys Ala Ile Val Asp Leu Leu Phe Lys Thr Asn Arg
945                 950                 955                 960

Lys Val Thr Val Lys Gln Leu Lys Glu Asp Tyr Phe Lys Lys Ile Glu
                965                 970                 975

Cys Phe Asp Ser Val Glu Ile Ser Gly Val Glu Asp Arg Phe Asn Ala
            980                 985                 990

Ser Leu Gly Thr Tyr His Asp Leu  Leu Lys Ile Ile Lys  Asp Lys Asp
        995                 1000                 1005

Phe Leu  Asp Asn Glu Glu Asn  Glu Asp Ile Leu Glu  Asp Ile Val
    1010                 1015                 1020

Leu Thr  Leu Thr Leu Phe Glu  Asp Arg Glu Met Ile  Glu Glu Arg
    1025                 1030                 1035

Leu Lys  Thr Tyr Ala His Leu  Phe Asp Asp Lys Val  Met Lys Gln
    1040                 1045                 1050

Leu Lys  Arg Arg Arg Tyr Thr  Gly Trp Gly Arg Leu  Ser Arg Lys
    1055                 1060                 1065

Leu Ile  Asn Gly Ile Arg Asp  Lys Gln Ser Gly Lys  Thr Ile Leu
    1070                 1075                 1080

Asp Phe  Leu Lys Ser Asp Gly  Phe Ala Asn Arg Asn  Phe Met Gln
    1085                 1090                 1095

Leu Ile  His Asp Asp Ser Leu  Thr Phe Lys Glu Asp  Ile Gln Lys
    1100                 1105                 1110

Ala Gln  Val Ser Gly Gln Gly  Asp Ser Leu His Glu  His Ile Ala
    1115                 1120                 1125

Asn Leu  Ala Gly Ser Pro Ala  Ile Lys Lys Gly Ile  Leu Gln Thr
    1130                 1135                 1140

Val Lys  Val Val Asp Glu Leu  Val Lys Val Met Gly  Arg His Lys
    1145                 1150                 1155

Pro Glu  Asn Ile Val Ile Glu  Met Ala Arg Glu Asn  Gln Thr Thr
    1160                 1165                 1170

Gln Lys  Gly Gln Lys Asn Ser  Arg Glu Arg Met Lys  Arg Ile Glu
    1175                 1180                 1185

Glu Gly  Ile Lys Glu Leu Gly  Ser Gln Ile Leu Lys  Glu His Pro

1190                1195                1200

Val Glu Asn Thr Gln Leu Gln Asn Glu Lys Leu Tyr Leu Tyr Tyr
    1205                1210                1215

Leu Gln Asn Gly Arg Asp Met Tyr Val Asp Gln Glu Leu Asp Ile
    1220                1225                1230

Asn Arg Leu Ser Asp Tyr Asp Val Asp His Ile Val Pro Gln Ser
    1235                1240                1245

Phe Leu Lys Asp Asp Ser Ile Asp Asn Lys Val Leu Thr Arg Ser
    1250                1255                1260

Asp Lys Asn Arg Gly Lys Ser Asp Asn Val Pro Ser Glu Glu Val
    1265                1270                1275

Val Lys Lys Met Lys Asn Tyr Trp Arg Gln Leu Leu Asn Ala Lys
    1280                1285                1290

Leu Ile Thr Gln Arg Lys Phe Asp Asn Leu Thr Lys Ala Glu Arg
    1295                1300                1305

Gly Gly Leu Ser Glu Leu Asp Lys Ala Gly Phe Ile Lys Arg Gln
    1310                1315                1320

Leu Val Glu Thr Arg Gln Ile Thr Lys His Val Ala Gln Ile Leu
    1325                1330                1335

Asp Ser Arg Met Asn Thr Lys Tyr Asp Glu Asn Asp Lys Leu Ile
    1340                1345                1350

Arg Glu Val Lys Val Ile Thr Leu Lys Ser Lys Leu Val Ser Asp
    1355                1360                1365

Phe Arg Lys Asp Phe Gln Phe Tyr Lys Val Arg Glu Ile Asn Asn
    1370                1375                1380

Tyr His His Ala His Asp Ala Tyr Leu Asn Ala Val Val Gly Thr
    1385                1390                1395

Ala Leu Ile Lys Lys Tyr Pro Lys Leu Glu Ser Glu Phe Val Tyr
    1400                1405                1410

Gly Asp Tyr Lys Val Tyr Asp Val Arg Lys Met Ile Ala Lys Ser
    1415                1420                1425

Glu Gln Glu Ile Gly Lys Ala Thr Ala Lys Tyr Phe Phe Tyr Ser
    1430                1435                1440

Asn Ile Met Asn Phe Phe Lys Thr Glu Ile Thr Leu Ala Asn Gly
    1445                1450                1455

Glu Ile Arg Lys Arg Pro Leu Ile Glu Thr Asn Gly Glu Thr Gly
    1460                1465                1470

Glu Ile Val Trp Asp Lys Gly Arg Asp Phe Ala Thr Val Arg Lys
    1475                1480                1485

Val Leu Ser Met Pro Gln Val Asn Ile Val Lys Lys Thr Glu Val
    1490                1495                1500

Gln Thr Gly Gly Phe Ser Lys Glu Ser Ile Leu Pro Lys Arg Asn
    1505                1510                1515

Ser Asp Lys Leu Ile Ala Arg Lys Lys Asp Trp Asp Pro Lys Lys
    1520                1525                1530

Tyr Gly Gly Phe Asp Ser Pro Thr Val Ala Tyr Ser Val Leu Val
    1535                1540                1545

Val Ala Lys Val Glu Lys Gly Lys Ser Lys Lys Leu Lys Ser Val
    1550                1555                1560

Lys Glu Leu Leu Gly Ile Thr Ile Met Glu Arg Ser Ser Phe Glu
    1565                1570                1575

Lys Asn Pro Ile Asp Phe Leu Glu Ala Lys Gly Tyr Lys Glu Val
    1580                1585                1590

Lys Lys Asp Leu Ile Ile Lys Leu Pro Lys Tyr Ser Leu Phe Glu
    1595                1600                1605

Leu Glu Asn Gly Arg Lys Arg Met Leu Ala Ser Ala Gly Glu Leu
    1610                1615                1620

Gln Lys Gly Asn Glu Leu Ala Leu Pro Ser Lys Tyr Val Asn Phe
    1625                1630                1635

Leu Tyr Leu Ala Ser His Tyr Glu Lys Leu Lys Gly Ser Pro Glu
    1640                1645                1650

Asp Asn Glu Gln Lys Gln Leu Phe Val Glu Gln His Lys His Tyr
    1655                1660                1665

Leu Asp Glu Ile Ile Glu Gln Ile Ser Glu Phe Ser Lys Arg Val
    1670                1675                1680

Ile Leu Ala Asp Ala Asn Leu Asp Lys Val Leu Ser Ala Tyr Asn
    1685                1690                1695

Lys His Arg Asp Lys Pro Ile Arg Glu Gln Ala Glu Asn Ile Ile
    1700                1705                1710

His Leu Phe Thr Leu Thr Asn Leu Gly Ala Pro Ala Ala Phe Lys
    1715                1720                1725

Tyr Phe Asp Thr Thr Ile Asp Arg Lys Arg Tyr Thr Ser Thr Lys
    1730                1735                1740

Glu Val Leu Asp Ala Thr Leu Ile His Gln Ser Ile Thr Gly Leu
    1745                1750                1755

Tyr Glu Thr Arg Ile Asp Leu Ser Gln Leu Gly Gly Asp Ser Gly
    1760                1765                1770

Gly Ser Lys Arg Thr Ala Asp Gly Ser Glu Phe Glu Pro Lys Lys
    1775                1780                1785

Lys Arg Lys Val
    1790

<210> 3
<211> 9
<212> PRT
<213> artificial sequence

<220>
<223> linker peptide

<400> 3

Gly Gly Ser Gly Gly Ser Gly Gly Ser
1               5

<210> 4
<211> 18
<212> PRT
<213> artificial sequence

<220>
<223> linker peptide

<400> 4

Gly Gly Ser Gly Gly Ser Gly Gly Ser Gly Gly Ser Gly Gly Ser Gly
1               5                   10                  15

Gly Ser

<210> 5
<211> 16
<212> PRT
<213> artificial sequence

<220>
<223> linker peptide

<400> 5

Ser Gly Ser Glu Thr Pro Gly Thr Ser Glu Ser Ala Thr Pro Glu Ser
1               5                   10                  15

<210> 6
<211> 32
<212> PRT
<213> artificial sequence

<220>
<223> linker peptide

<400> 6

Ser Gly Gly Ser Ser Gly Gly Ser Ser Gly Ser Glu Thr Pro Gly Thr
1               5                   10                  15

Ser Glu Ser Ala Thr Pro Glu Ser Ser Gly Gly Ser Ser Gly Gly Ser
            20                  25                  30

<210> 7
<211> 48
<212> PRT
<213> artificial sequence

<220>
<223> linker peptide

<400> 7

Ser Gly Gly Ser Ser Gly Gly Ser Ser Gly Ser Glu Thr Pro Gly Thr
1               5                   10                  15

Ser Glu Ser Ala Thr Pro Glu Ser Ser Gly Ser Glu Thr Pro Gly Thr
            20                  25                  30

Ser Glu Ser Ala Thr Pro Glu Ser Ser Gly Gly Ser Ser Gly Gly Ser
        35                  40                  45

<210> 8
<211> 1567
<212> PRT

<213>  artificial sequence

<220>
<223>  fusion protein

<400>  8

Met Ser Asp Lys Pro Lys Arg Pro Leu Ser Ala Tyr Met Leu Trp Leu
1               5                   10                  15

Asn Ser Ala Arg Glu Ser Ile Lys Arg Glu Asn Pro Gly Ile Lys Val
            20                  25                  30

Thr Glu Val Ala Lys Arg Gly Gly Glu Leu Trp Arg Ala Met Lys Asp
        35                  40                  45

Lys Ser Glu Trp Glu Ala Lys Ala Ala Lys Ala Lys Asp Asp Tyr Asp
    50                  55                  60

Arg Ala Val Lys Glu Phe Glu Ala Asn Gly Gly Ser Ser Ala Ala Asn
65                  70                  75                  80

Gly Gly Gly Ala Lys Lys Arg Ala Lys Pro Ala Lys Lys Val Ala Lys
                85                  90                  95

Lys Ser Lys Lys Glu Glu Ser Asp Glu Asp Asp Asp Asp Glu Ser Glu
            100                 105                 110

Ser Gly Gly Ser Ser Gly Gly Ser Ser Gly Ser Glu Thr Pro Gly Thr
        115                 120                 125

Ser Glu Ser Ala Thr Pro Glu Ser Ser Gly Gly Ser Ser Gly Gly Ser
    130                 135                 140

Met Asp Tyr Lys Asp His Asp Gly Asp Tyr Lys Asp His Asp Ile Asp
145                 150                 155                 160

Tyr Lys Asp Asp Asp Asp Lys Met Ala Pro Lys Lys Lys Arg Lys Val
                165                 170                 175

Gly Ile His Gly Val Pro Ala Ala Asp Lys Lys Tyr Ser Ile Gly Leu
            180                 185                 190

Asp Ile Gly Thr Asn Ser Val Gly Trp Ala Val Ile Thr Asp Glu Tyr
        195                 200                 205

Lys Val Pro Ser Lys Lys Phe Lys Val Leu Gly Asn Thr Asp Arg His
    210                 215                 220

Ser Ile Lys Lys Asn Leu Ile Gly Ala Leu Leu Phe Asp Ser Gly Glu
225                 230                 235                 240

Thr Ala Glu Ala Thr Arg Leu Lys Arg Thr Ala Arg Arg Arg Tyr Thr
                245                 250                 255

Arg Arg Lys Asn Arg Ile Cys Tyr Leu Gln Glu Ile Phe Ser Asn Glu
            260                 265                 270

Met Ala Lys Val Asp Asp Ser Phe Phe His Arg Leu Glu Glu Ser Phe
        275                 280                 285

Leu Val Glu Glu Asp Lys Lys His Glu Arg His Pro Ile Phe Gly Asn
    290                 295                 300

Ile Val Asp Glu Val Ala Tyr His Glu Lys Tyr Pro Thr Ile Tyr His
305                 310                 315                 320

Leu Arg Lys Lys Leu Val Asp Ser Thr Asp Lys Ala Asp Leu Arg Leu
                325                 330                 335

Ile Tyr Leu Ala Leu Ala His Met Ile Lys Phe Arg Gly His Phe Leu
            340                 345                 350

Ile Glu Gly Asp Leu Asn Pro Asp Asn Ser Asp Val Asp Lys Leu Phe
        355                 360                 365

Ile Gln Leu Val Gln Thr Tyr Asn Gln Leu Phe Glu Glu Asn Pro Ile
    370                 375                 380

Asn Ala Ser Gly Val Asp Ala Lys Ala Ile Leu Ser Ala Arg Leu Ser
385                 390                 395                 400

Lys Ser Arg Arg Leu Glu Asn Leu Ile Ala Gln Leu Pro Gly Glu Lys
                405                 410                 415

Lys Asn Gly Leu Phe Gly Asn Leu Ile Ala Leu Ser Leu Gly Leu Thr
            420                 425                 430

Pro Asn Phe Lys Ser Asn Phe Asp Leu Ala Glu Asp Ala Lys Leu Gln
        435                 440                 445

Leu Ser Lys Asp Thr Tyr Asp Asp Asp Leu Asp Asn Leu Leu Ala Gln
    450                 455                 460

Ile Gly Asp Gln Tyr Ala Asp Leu Phe Leu Ala Ala Lys Asn Leu Ser
465                 470                 475                 480

Asp Ala Ile Leu Leu Ser Asp Ile Leu Arg Val Asn Thr Glu Ile Thr
                485                 490                 495

Lys Ala Pro Leu Ser Ala Ser Met Ile Lys Arg Tyr Asp Glu His His
            500                 505                 510

Gln Asp Leu Thr Leu Leu Lys Ala Leu Val Arg Gln Gln Leu Pro Glu
        515                 520                 525

Lys Tyr Lys Glu Ile Phe Phe Asp Gln Ser Lys Asn Gly Tyr Ala Gly
    530                 535                 540

Tyr Ile Asp Gly Gly Ala Ser Gln Glu Glu Phe Tyr Lys Phe Ile Lys
545                 550                 555                 560

Pro Ile Leu Glu Lys Met Asp Gly Thr Glu Glu Leu Leu Val Lys Leu
                565                 570                 575

Asn Arg Glu Asp Leu Leu Arg Lys Gln Arg Thr Phe Asp Asn Gly Ser
            580                 585                 590

Ile Pro His Gln Ile His Leu Gly Glu Leu His Ala Ile Leu Arg Arg
        595                 600                 605

Gln Glu Asp Phe Tyr Pro Phe Leu Lys Asp Asn Arg Glu Lys Ile Glu
    610                 615                 620

Lys Ile Leu Thr Phe Arg Ile Pro Tyr Tyr Val Gly Pro Leu Ala Arg
625                 630                 635                 640

Gly Asn Ser Arg Phe Ala Trp Met Thr Arg Lys Ser Glu Glu Thr Ile
                645                 650                 655

Thr Pro Trp Asn Phe Glu Glu Val Val Asp Lys Gly Ala Ser Ala Gln
            660                 665                 670

Ser Phe Ile Glu Arg Met Thr Asn Phe Asp Lys Asn Leu Pro Asn Glu
        675                 680                 685

Lys Val Leu Pro Lys His Ser Leu Leu Tyr Glu Tyr Phe Thr Val Tyr
    690                 695                 700

Asn Glu Leu Thr Lys Val Lys Tyr Val Thr Glu Gly Met Arg Lys Pro
705                 710                 715                 720

Ala Phe Leu Ser Gly Glu Gln Lys Lys Ala Ile Val Asp Leu Leu Phe
                725                 730                 735

Lys Thr Asn Arg Lys Val Thr Val Lys Gln Leu Lys Glu Asp Tyr Phe
            740                 745                 750

Lys Lys Ile Glu Cys Phe Asp Ser Val Glu Ile Ser Gly Val Glu Asp
        755                 760                 765

Arg Phe Asn Ala Ser Leu Gly Thr Tyr His Asp Leu Leu Lys Ile Ile
    770                 775                 780

Lys Asp Lys Asp Phe Leu Asp Asn Glu Glu Asn Glu Asp Ile Leu Glu
785                 790                 795                 800

Asp Ile Val Leu Thr Leu Thr Leu Phe Glu Asp Arg Glu Met Ile Glu
                805                 810                 815

Glu Arg Leu Lys Thr Tyr Ala His Leu Phe Asp Asp Lys Val Met Lys
            820                 825                 830

Gln Leu Lys Arg Arg Arg Tyr Thr Gly Trp Gly Arg Leu Ser Arg Lys
        835                 840                 845

Leu Ile Asn Gly Ile Arg Asp Lys Gln Ser Gly Lys Thr Ile Leu Asp
    850                 855                 860

Phe Leu Lys Ser Asp Gly Phe Ala Asn Arg Asn Phe Met Gln Leu Ile
865                 870                 875                 880

His Asp Asp Ser Leu Thr Phe Lys Glu Asp Ile Gln Lys Ala Gln Val
                885                 890                 895

Ser Gly Gln Gly Asp Ser Leu His Glu His Ile Ala Asn Leu Ala Gly
            900                 905                 910

Ser Pro Ala Ile Lys Lys Gly Ile Leu Gln Thr Val Lys Val Val Asp
        915                 920                 925

Glu Leu Val Lys Val Met Gly Arg His Lys Pro Glu Asn Ile Val Ile
    930                 935                 940

Glu Met Ala Arg Glu Asn Gln Thr Thr Gln Lys Gly Gln Lys Asn Ser
945                 950                 955                 960

Arg Glu Arg Met Lys Arg Ile Glu Glu Gly Ile Lys Glu Leu Gly Ser
                965                 970                 975

Gln Ile Leu Lys Glu His Pro Val Glu Asn Thr Gln Leu Gln Asn Glu

            980                 985                 990

Lys Leu Tyr Leu Tyr Tyr Leu Gln Asn Gly Arg Asp Met Tyr Val Asp
        995                 1000                1005

Gln Glu Leu Asp Ile Asn Arg Leu Ser Asp Tyr Asp Val Asp His
    1010                1015                1020

Ile Val Pro Gln Ser Phe Leu Lys Asp Asp Ser Ile Asp Asn Lys
    1025                1030                1035

Val Leu Thr Arg Ser Asp Lys Asn Arg Gly Lys Ser Asp Asn Val
    1040                1045                1050

Pro Ser Glu Glu Val Val Lys Lys Met Lys Asn Tyr Trp Arg Gln
    1055                1060                1065

Leu Leu Asn Ala Lys Leu Ile Thr Gln Arg Lys Phe Asp Asn Leu
    1070                1075                1080

Thr Lys Ala Glu Arg Gly Gly Leu Ser Glu Leu Asp Lys Ala Gly
    1085                1090                1095

Phe Ile Lys Arg Gln Leu Val Glu Thr Arg Gln Ile Thr Lys His
    1100                1105                1110

Val Ala Gln Ile Leu Asp Ser Arg Met Asn Thr Lys Tyr Asp Glu
    1115                1120                1125

Asn Asp Lys Leu Ile Arg Glu Val Lys Val Ile Thr Leu Lys Ser
    1130                1135                1140

Lys Leu Val Ser Asp Phe Arg Lys Asp Phe Gln Phe Tyr Lys Val
    1145                1150                1155

Arg Glu Ile Asn Asn Tyr His His Ala His Asp Ala Tyr Leu Asn
    1160                1165                1170

Ala Val Val Gly Thr Ala Leu Ile Lys Lys Tyr Pro Lys Leu Glu
    1175                1180                1185

Ser Glu Phe Val Tyr Gly Asp Tyr Lys Val Tyr Asp Val Arg Lys
    1190                1195                1200

Met Ile Ala Lys Ser Glu Gln Glu Ile Gly Lys Ala Thr Ala Lys
    1205                1210                1215

Tyr Phe Phe Tyr Ser Asn Ile Met Asn Phe Phe Lys Thr Glu Ile
    1220                1225                1230

Thr Leu Ala Asn Gly Glu Ile Arg Lys Arg Pro Leu Ile Glu Thr
    1235                1240                1245

Asn Gly Glu Thr Gly Glu Ile Val Trp Asp Lys Gly Arg Asp Phe
    1250                1255                1260

Ala Thr Val Arg Lys Val Leu Ser Met Pro Gln Val Asn Ile Val
    1265                1270                1275

Lys Lys Thr Glu Val Gln Thr Gly Gly Phe Ser Lys Glu Ser Ile
    1280                1285                1290

Leu Pro Lys Arg Asn Ser Asp Lys Leu Ile Ala Arg Lys Lys Asp
    1295                1300                1305

Trp Asp Pro Lys Lys Tyr Gly Gly Phe Asp Ser Pro Thr Val Ala
    1310                1315                1320

Tyr Ser Val Leu Val Val Ala Lys Val Glu Lys Gly Lys Ser Lys
    1325                1330                1335

Lys Leu Lys Ser Val Lys Glu Leu Leu Gly Ile Thr Ile Met Glu
    1340                1345                1350

Arg Ser Ser Phe Glu Lys Asn Pro Ile Asp Phe Leu Glu Ala Lys
    1355                1360                1365

Gly Tyr Lys Glu Val Lys Lys Asp Leu Ile Ile Lys Leu Pro Lys
    1370                1375                1380

Tyr Ser Leu Phe Glu Leu Glu Asn Gly Arg Lys Arg Met Leu Ala
    1385                1390                1395

Ser Ala Gly Glu Leu Gln Lys Gly Asn Glu Leu Ala Leu Pro Ser
    1400                1405                1410

Lys Tyr Val Asn Phe Leu Tyr Leu Ala Ser His Tyr Glu Lys Leu
    1415                1420                1425

Lys Gly Ser Pro Glu Asp Asn Glu Gln Lys Gln Leu Phe Val Glu
    1430                1435                1440

Gln His Lys His Tyr Leu Asp Glu Ile Ile Glu Gln Ile Ser Glu
    1445                1450                1455

Phe Ser Lys Arg Val Ile Leu Ala Asp Ala Asn Leu Asp Lys Val
    1460                1465                1470

Leu Ser Ala Tyr Asn Lys His Arg Asp Lys Pro Ile Arg Glu Gln
    1475                1480                1485

Ala Glu Asn Ile Ile His Leu Phe Thr Leu Thr Asn Leu Gly Ala
    1490                1495                1500

Pro Ala Ala Phe Lys Tyr Phe Asp Thr Thr Ile Asp Arg Lys Arg
    1505                1510                1515

Tyr Thr Ser Thr Lys Glu Val Leu Asp Ala Thr Leu Ile His Gln
    1520                1525                1530

Ser Ile Thr Gly Leu Tyr Glu Thr Arg Ile Asp Leu Ser Gln Leu
    1535                1540                1545

Gly Gly Asp Lys Arg Pro Ala Ala Thr Lys Lys Ala Gly Gln Ala
    1550                1555                1560

Lys Lys Lys Lys
    1565

<210> 9
<211> 1952
<212> PRT
<213> artificial sequence

<220>
<223> fusion protein

<400> 9

Met Pro Lys Lys Lys Arg Lys Val Ser Glu Val Glu Phe Ser His Glu
1               5                   10                  15

Tyr Trp Met Arg His Ala Leu Thr Leu Ala Lys Arg Ala Trp Asp Glu
            20                  25                  30

Arg Glu Val Pro Val Gly Ala Val Leu Val His Asn Asn Arg Val Ile
        35                  40                  45

Gly Glu Gly Trp Asn Arg Pro Ile Gly Arg His Asp Pro Thr Ala His
    50                  55                  60

Ala Glu Ile Met Ala Leu Arg Gln Gly Gly Leu Val Met Gln Asn Tyr
65                  70                  75                  80

Arg Leu Ile Asp Ala Thr Leu Tyr Val Thr Leu Glu Pro Cys Val Met
                85                  90                  95

Cys Ala Gly Ala Met Ile His Ser Arg Ile Gly Arg Val Val Phe Gly
            100                 105                 110

Ala Arg Asp Ala Lys Thr Gly Ala Ala Gly Ser Leu Met Asp Val Leu
        115                 120                 125

His His Pro Gly Met Asn His Arg Val Glu Ile Thr Glu Gly Ile Leu
    130                 135                 140

Ala Asp Glu Cys Ala Ala Leu Leu Ser Asp Phe Phe Arg Met Arg Arg
145                 150                 155                 160

Gln Glu Ile Lys Ala Gln Lys Lys Ala Gln Ser Ser Thr Asp Ser Gly
                165                 170                 175

Gly Ser Ser Gly Gly Ser Ser Gly Ser Glu Thr Pro Gly Thr Ser Glu
            180                 185                 190

Ser Ala Thr Pro Glu Ser Ser Gly Gly Ser Ser Gly Gly Ser Ser Glu
        195                 200                 205

Val Glu Phe Ser His Glu Tyr Trp Met Arg His Ala Leu Thr Leu Ala
    210                 215                 220

Lys Arg Ala Arg Asp Glu Arg Glu Val Pro Val Gly Ala Val Leu Val
225                 230                 235                 240

Leu Asn Asn Arg Val Ile Gly Glu Gly Trp Asn Arg Ala Ile Gly Leu
                245                 250                 255

His Asp Pro Thr Ala His Ala Glu Ile Met Ala Leu Arg Gln Gly Gly
            260                 265                 270

Leu Val Met Gln Asn Tyr Arg Leu Ile Asp Ala Thr Leu Tyr Val Thr
        275                 280                 285

Phe Glu Pro Cys Val Met Cys Ala Gly Ala Met Ile His Ser Arg Ile
    290                 295                 300

Gly Arg Val Val Phe Gly Val Arg Asn Ala Lys Thr Gly Ala Ala Gly
305                 310                 315                 320

Ser Leu Met Asp Val Leu His Tyr Pro Gly Met Asn His Arg Val Glu
                325                 330                 335

Ile Thr Glu Gly Ile Leu Ala Asp Glu Cys Ala Ala Leu Leu Cys Tyr
            340                 345                 350

Phe Phe Arg Met Pro Arg Gln Val Phe Asn Ala Gln Lys Lys Ala Gln
        355                 360                 365

Ser Ser Thr Asp Ser Gly Gly Ser Ser Gly Gly Ser Ser Gly Ser Glu
    370                 375                 380

Thr Pro Gly Thr Ser Glu Ser Ala Thr Pro Glu Ser Ser Gly Gly Ser
385                 390                 395                 400

Ser Gly Gly Ser Asp Lys Lys Tyr Ser Ile Gly Leu Ala Ile Gly Thr
                405                 410                 415

Asn Ser Val Gly Trp Ala Val Ile Thr Asp Glu Tyr Lys Val Pro Ser
            420                 425                 430

Lys Lys Phe Lys Val Leu Gly Asn Thr Asp Arg His Ser Ile Lys Lys
        435                 440                 445

Asn Leu Ile Gly Ala Leu Leu Phe Asp Ser Gly Glu Thr Ala Glu Ala
    450                 455                 460

Thr Arg Leu Lys Arg Thr Ala Arg Arg Arg Tyr Thr Arg Arg Lys Asn
465                 470                 475                 480

Arg Ile Cys Tyr Leu Gln Glu Ile Phe Ser Asn Glu Met Ala Lys Val
                485                 490                 495

Asp Asp Ser Phe Phe His Arg Leu Glu Glu Ser Phe Leu Val Glu Glu
            500                 505                 510

Asp Lys Lys His Glu Arg His Pro Ile Phe Gly Asn Ile Val Asp Glu
        515                 520                 525

Val Ala Tyr His Glu Lys Tyr Pro Thr Ile Tyr His Leu Arg Lys Lys
    530                 535                 540

Leu Val Asp Ser Thr Asp Lys Ala Asp Leu Arg Leu Ile Tyr Leu Ala
545                 550                 555                 560

Leu Ala His Met Ile Lys Phe Arg Gly His Phe Leu Ile Glu Gly Asp
                565                 570                 575

Leu Asn Pro Asp Asn Ser Asp Val Asp Lys Leu Phe Ile Gln Leu Val
            580                 585                 590

Gln Thr Tyr Asn Gln Leu Phe Glu Glu Asn Pro Ile Asn Ala Ser Gly
        595                 600                 605

Val Asp Ala Lys Ala Ile Leu Ser Ala Arg Leu Ser Lys Ser Arg Arg
    610                 615                 620

Leu Glu Asn Leu Ile Ala Gln Leu Pro Gly Glu Lys Lys Asn Gly Leu
625                 630                 635                 640

Phe Gly Asn Leu Ile Ala Leu Ser Leu Gly Leu Thr Pro Asn Phe Lys
                645                 650                 655

Ser Asn Phe Asp Leu Ala Glu Asp Ala Lys Leu Gln Leu Ser Lys Asp
            660                 665                 670

Thr Tyr Asp Asp Asp Leu Asp Asn Leu Leu Ala Gln Ile Gly Asp Gln
        675                 680                 685

Tyr Ala Asp Leu Phe Leu Ala Ala Lys Asn Leu Ser Asp Ala Ile Leu
    690                 695                 700

Leu Ser Asp Ile Leu Arg Val Asn Thr Glu Ile Thr Lys Ala Pro Leu
705                 710                 715                 720

Ser Ala Ser Met Ile Lys Arg Tyr Asp Glu His His Gln Asp Leu Thr
                725                 730                 735

Leu Leu Lys Ala Leu Val Arg Gln Gln Leu Pro Glu Lys Tyr Lys Glu
            740                 745                 750

Ile Phe Phe Asp Gln Ser Lys Asn Gly Tyr Ala Gly Tyr Ile Asp Gly
        755                 760                 765

Gly Ala Ser Gln Glu Glu Phe Tyr Lys Phe Ile Lys Pro Ile Leu Glu
    770                 775                 780

Lys Met Asp Gly Thr Glu Glu Leu Leu Val Lys Leu Asn Arg Glu Asp
785                 790                 795                 800

Leu Leu Arg Lys Gln Arg Thr Phe Asp Asn Gly Ser Ile Pro His Gln
                805                 810                 815

Ile His Leu Gly Glu Leu His Ala Ile Leu Arg Arg Gln Glu Asp Phe
            820                 825                 830

Tyr Pro Phe Leu Lys Asp Asn Arg Glu Lys Ile Glu Lys Ile Leu Thr

835                 840                 845

Phe Arg Ile Pro Tyr Tyr Val Gly Pro Leu Ala Arg Gly Asn Ser Arg
    850                 855                 860

Phe Ala Trp Met Thr Arg Lys Ser Glu Glu Thr Ile Thr Pro Trp Asn
865                 870                 875                 880

Phe Glu Glu Val Val Asp Lys Gly Ala Ser Ala Gln Ser Phe Ile Glu
                885                 890                 895

Arg Met Thr Asn Phe Asp Lys Asn Leu Pro Asn Glu Lys Val Leu Pro
            900                 905                 910

Lys His Ser Leu Leu Tyr Glu Tyr Phe Thr Val Tyr Asn Glu Leu Thr
        915                 920                 925

Lys Val Lys Tyr Val Thr Glu Gly Met Arg Lys Pro Ala Phe Leu Ser
    930                 935                 940

Gly Glu Gln Lys Lys Ala Ile Val Asp Leu Leu Phe Lys Thr Asn Arg
945                 950                 955                 960

Lys Val Thr Val Lys Gln Leu Lys Glu Asp Tyr Phe Lys Lys Ile Glu
                965                 970                 975

Cys Phe Asp Ser Val Glu Ile Ser Gly Val Glu Asp Arg Phe Asn Ala
            980                 985                 990

Ser Leu Gly Thr Tyr His Asp Leu  Leu Lys Ile Ile Lys  Asp Lys Asp
        995                 1000                1005

Phe Leu  Asp Asn Glu Glu Asn  Glu Asp Ile Leu Glu  Asp Ile Val
    1010                1015                1020

Leu Thr  Leu Thr Leu Phe Glu  Asp Arg Glu Met Ile  Glu Glu Arg
    1025                1030                1035

Leu Lys  Thr Tyr Ala His Leu  Phe Asp Asp Lys Val  Met Lys Gln
    1040                1045                1050

Leu Lys  Arg Arg Arg Tyr Thr  Gly Trp Gly Arg Leu  Ser Arg Lys
    1055                1060                1065

Leu Ile  Asn Gly Ile Arg Asp  Lys Gln Ser Gly Lys  Thr Ile Leu
    1070                1075                1080

Asp Phe Leu Lys Ser Asp Gly Phe Ala Asn Arg Asn Phe Met Gln
    1085                1090                1095

Leu Ile His Asp Asp Ser Leu Thr Phe Lys Glu Asp Ile Gln Lys
    1100                1105                1110

Ala Gln Val Ser Gly Gln Gly Asp Ser Leu His Glu His Ile Ala
    1115                1120                1125

Asn Leu Ala Gly Ser Pro Ala Ile Lys Lys Gly Ile Leu Gln Thr
    1130                1135                1140

Val Lys Val Val Asp Glu Leu Val Lys Val Met Gly Arg His Lys
    1145                1150                1155

Pro Glu Asn Ile Val Ile Glu Met Ala Arg Glu Asn Gln Thr Thr
    1160                1165                1170

Gln Lys Gly Gln Lys Asn Ser Arg Glu Arg Met Lys Arg Ile Glu
    1175                1180                1185

Glu Gly Ile Lys Glu Leu Gly Ser Gln Ile Leu Lys Glu His Pro
    1190                1195                1200

Val Glu Asn Thr Gln Leu Gln Asn Glu Lys Leu Tyr Leu Tyr Tyr
    1205                1210                1215

Leu Gln Asn Gly Arg Asp Met Tyr Val Asp Gln Glu Leu Asp Ile
    1220                1225                1230

Asn Arg Leu Ser Asp Tyr Asp Val Asp His Ile Val Pro Gln Ser
    1235                1240                1245

Phe Leu Lys Asp Asp Ser Ile Asp Asn Lys Val Leu Thr Arg Ser
    1250                1255                1260

Asp Lys Asn Arg Gly Lys Ser Asp Asn Val Pro Ser Glu Glu Val
    1265                1270                1275

Val Lys Lys Met Lys Asn Tyr Trp Arg Gln Leu Leu Asn Ala Lys
    1280                1285                1290

Leu Ile Thr Gln Arg Lys Phe Asp Asn Leu Thr Lys Ala Glu Arg
    1295                1300                1305

Gly Gly Leu Ser Glu Leu Asp Lys Ala Gly Phe Ile Lys Arg Gln
    1310                1315                1320

Leu Val Glu Thr Arg Gln Ile Thr Lys His Val Ala Gln Ile Leu
    1325                1330                1335

Asp Ser Arg Met Asn Thr Lys Tyr Asp Glu Asn Asp Lys Leu Ile
    1340                1345                1350

Arg Glu Val Lys Val Ile Thr Leu Lys Ser Lys Leu Val Ser Asp
    1355                1360                1365

Phe Arg Lys Asp Phe Gln Phe Tyr Lys Val Arg Glu Ile Asn Asn
    1370                1375                1380

Tyr His His Ala His Asp Ala Tyr Leu Asn Ala Val Val Gly Thr
    1385                1390                1395

Ala Leu Ile Lys Lys Tyr Pro Lys Leu Glu Ser Glu Phe Val Tyr
    1400                1405                1410

Gly Asp Tyr Lys Val Tyr Asp Val Arg Lys Met Ile Ala Lys Ser
    1415                1420                1425

Glu Gln Glu Ile Gly Lys Ala Thr Ala Lys Tyr Phe Phe Tyr Ser
    1430                1435                1440

Asn Ile Met Asn Phe Phe Lys Thr Glu Ile Thr Leu Ala Asn Gly
    1445                1450                1455

Glu Ile Arg Lys Arg Pro Leu Ile Glu Thr Asn Gly Glu Thr Gly
    1460                1465                1470

Glu Ile Val Trp Asp Lys Gly Arg Asp Phe Ala Thr Val Arg Lys
    1475                1480                1485

Val Leu Ser Met Pro Gln Val Asn Ile Val Lys Lys Thr Glu Val
    1490                1495                1500

Gln Thr Gly Gly Phe Ser Lys Glu Ser Ile Leu Pro Lys Arg Asn
    1505                1510                1515

Ser Asp Lys Leu Ile Ala Arg Lys Lys Asp Trp Asp Pro Lys Lys
    1520                1525                1530

Tyr Gly Gly Phe Asp Ser Pro Thr Val Ala Tyr Ser Val Leu Val
    1535                1540                1545

Val Ala Lys Val Glu Lys Gly Lys Ser Lys Lys Leu Lys Ser Val
    1550                1555                1560

Lys Glu Leu Leu Gly Ile Thr Ile Met Glu Arg Ser Ser Phe Glu
    1565                1570                1575

Lys Asn Pro Ile Asp Phe Leu Glu Ala Lys Gly Tyr Lys Glu Val
    1580                1585                1590

Lys Lys Asp Leu Ile Ile Lys Leu Pro Lys Tyr Ser Leu Phe Glu
    1595                1600                1605

Leu Glu Asn Gly Arg Lys Arg Met Leu Ala Ser Ala Gly Glu Leu
    1610                1615                1620

Gln Lys Gly Asn Glu Leu Ala Leu Pro Ser Lys Tyr Val Asn Phe
    1625                1630                1635

Leu Tyr Leu Ala Ser His Tyr Glu Lys Leu Lys Gly Ser Pro Glu
    1640                1645                1650

Asp Asn Glu Gln Lys Gln Leu Phe Val Glu Gln His Lys His Tyr
    1655                1660                1665

Leu Asp Glu Ile Ile Glu Gln Ile Ser Glu Phe Ser Lys Arg Val
    1670                1675                1680

Ile Leu Ala Asp Ala Asn Leu Asp Lys Val Leu Ser Ala Tyr Asn
    1685                1690                1695

Lys His Arg Asp Lys Pro Ile Arg Glu Gln Ala Glu Asn Ile Ile
    1700                1705                1710

His Leu Phe Thr Leu Thr Asn Leu Gly Ala Pro Ala Ala Phe Lys
    1715                1720                1725

Tyr Phe Asp Thr Thr Ile Asp Arg Lys Arg Tyr Thr Ser Thr Lys
    1730                1735                1740

Glu Val Leu Asp Ala Thr Leu Ile His Gln Ser Ile Thr Gly Leu
    1745                1750                1755

Tyr Glu Thr Arg Ile Asp Leu Ser Gln Leu Gly Gly Asp Ser Gly
    1760                1765                1770

Gly Ser Lys Arg Thr Ala Asp Gly Ser Glu Phe Glu Pro Lys Lys
    1775                1780                1785

Lys Arg Lys Val Ser Gly Gly Ser Ser Gly Gly Ser Ser Gly Ser

1790                    1795                    1800

Glu Thr  Pro Gly Thr Ser Glu  Ser Ala Thr Pro Glu  Ser Ser Gly
    1805                    1810                    1815

Ser Glu  Thr Pro Gly Thr Ser  Glu Ser Ala Thr Pro  Glu Ser Ser
    1820                    1825                    1830

Gly Gly  Ser Ser Gly Gly Ser  Met Ser Asp Lys Pro  Lys Arg Pro
    1835                    1840                    1845

Leu Ser  Ala Tyr Met Leu Trp  Leu Asn Ser Ala Arg  Glu Ser Ile
    1850                    1855                    1860

Lys Arg  Glu Asn Pro Gly Ile  Lys Val Thr Glu Val  Ala Lys Arg
    1865                    1870                    1875

Gly Gly  Glu Leu Trp Arg Ala  Met Lys Asp Lys Ser  Glu Trp Glu
    1880                    1885                    1890

Ala Lys  Ala Ala Lys Ala Lys  Asp Asp Tyr Asp Arg  Ala Val Lys
    1895                    1900                    1905

Glu Phe  Glu Ala Asn Gly Gly  Ser Ser Ala Ala Asn  Gly Gly Gly
    1910                    1915                    1920

Ala Lys  Lys Arg Ala Lys Pro  Ala Lys Lys Val Ala  Lys Lys Ser
    1925                    1930                    1935

Lys Lys  Glu Glu Ser Asp Glu  Asp Asp Asp Asp Glu  Ser Glu
    1940                    1945                    1950

<210>  10
<211>  112
<212>  PRT
<213>  artificial sequence

<220>
<223>   DNA double-strand binding domain

<400>  10

Met Ser Asp Lys Pro Lys Arg Pro Leu Ser Ala Tyr Met Leu Trp Leu
1               5                   10                  15

Asn Ser Ala Arg Glu Ser Ile Lys Arg Glu Asn Pro Gly Ile Lys Val
            20                  25                  30

Thr Glu Val Ala Lys Arg Gly Gly Glu Leu Trp Arg Ala Met Lys Asp
        35                  40                  45

Lys Ser Glu Trp Glu Ala Lys Ala Ala Lys Ala Lys Asp Asp Tyr Asp
    50                  55                  60

Arg Ala Val Lys Glu Phe Glu Ala Asn Gly Gly Ser Ser Ala Ala Asn
65                  70                  75                  80

Gly Gly Gly Ala Lys Lys Arg Ala Lys Pro Ala Lys Lys Val Ala Lys
                85                  90                  95

Lys Ser Lys Lys Glu Glu Ser Asp Glu Asp Asp Asp Asp Glu Ser Glu
            100                 105                 110

<210>  11
<211>  66
<212>  PRT
<213>  artificial sequence

<220>
<223>   DNA double-strand binding domain

<400>  11

Met Val Lys Val Lys Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Asp Met Leu Ala Arg Ala Glu Arg Glu
    50                  55                  60

Lys Lys
65

<210>  12
<211>  1667
<212>  PRT
<213>  artificial sequence

<220>
<223>    base editor element

<400>  12

Met Lys Arg Thr Ala Asp Gly Ser Glu Phe Glu Ser Pro Lys Lys Lys
1               5                   10                  15

Arg Lys Val Ser Ser Glu Thr Gly Pro Val Ala Val Asp Pro Thr Leu

            20                  25                  30

Arg Arg Arg Ile Glu Pro His Glu Phe Glu Val Phe Phe Asp Pro Arg
        35                  40                  45

Glu Leu Arg Lys Glu Thr Cys Leu Leu Tyr Glu Ile Asn Trp Gly Gly
    50                  55                  60

Arg His Ser Ile Trp Arg His Thr Ser Gln Asn Thr Asn Lys His Val
65                  70                  75                  80

Glu Val Asn Phe Ile Glu Lys Phe Thr Thr Glu Arg Tyr Phe Cys Pro
                85                  90                  95

Asn Thr Arg Cys Ser Ile Thr Trp Phe Leu Ser Trp Ser Pro Cys Gly
            100                 105                 110

Glu Cys Ser Arg Ala Ile Thr Glu Phe Leu Ser Arg Tyr Pro His Val
        115                 120                 125

Thr Leu Phe Ile Tyr Ile Ala Arg Leu Tyr His His Ala Asp Pro Arg
    130                 135                 140

Asn Arg Gln Gly Leu Arg Asp Leu Ile Ser Ser Gly Val Thr Ile Gln
145                 150                 155                 160

Ile Met Thr Glu Gln Glu Ser Gly Tyr Cys Trp Arg Asn Phe Val Asn
                165                 170                 175

Tyr Ser Pro Ser Asn Glu Ala His Trp Pro Arg Tyr Pro His Leu Trp
            180                 185                 190

Val Arg Leu Tyr Val Leu Glu Leu Tyr Cys Ile Ile Leu Gly Leu Pro
        195                 200                 205

Pro Cys Leu Asn Ile Leu Arg Arg Lys Gln Pro Gln Leu Thr Phe Phe
    210                 215                 220

Thr Ile Ala Leu Gln Ser Cys His Tyr Gln Arg Leu Pro Pro His Ile
225                 230                 235                 240

Leu Trp Ala Thr Gly Leu Lys Ser Gly Gly Ser Ser Gly Gly Ser Ser
                245                 250                 255

Gly Ser Glu Thr Pro Gly Thr Ser Glu Ser Ala Thr Pro Glu Ser Ser
            260                 265                 270

Gly Gly Ser Ser Gly Gly Ser Asp Lys Lys Tyr Ser Ile Gly Leu Ala
        275                 280                 285

Ile Gly Thr Asn Ser Val Gly Trp Ala Val Ile Thr Asp Glu Tyr Lys
    290                 295                 300

Val Pro Ser Lys Lys Phe Lys Val Leu Gly Asn Thr Asp Arg His Ser
305                 310                 315                 320

Ile Lys Lys Asn Leu Ile Gly Ala Leu Leu Phe Asp Ser Gly Glu Thr
                325                 330                 335

Ala Glu Ala Thr Arg Leu Lys Arg Thr Ala Arg Arg Arg Tyr Thr Arg
            340                 345                 350

Arg Lys Asn Arg Ile Cys Tyr Leu Gln Glu Ile Phe Ser Asn Glu Met
        355                 360                 365

Ala Lys Val Asp Asp Ser Phe Phe His Arg Leu Glu Glu Ser Phe Leu
    370                 375                 380

Val Glu Glu Asp Lys Lys His Glu Arg His Pro Ile Phe Gly Asn Ile
385                 390                 395                 400

Val Asp Glu Val Ala Tyr His Glu Lys Tyr Pro Thr Ile Tyr His Leu
                405                 410                 415

Arg Lys Lys Leu Val Asp Ser Thr Asp Lys Ala Asp Leu Arg Leu Ile
            420                 425                 430

Tyr Leu Ala Leu Ala His Met Ile Lys Phe Arg Gly His Phe Leu Ile
        435                 440                 445

Glu Gly Asp Leu Asn Pro Asp Asn Ser Asp Val Asp Lys Leu Phe Ile
    450                 455                 460

Gln Leu Val Gln Thr Tyr Asn Gln Leu Phe Glu Glu Asn Pro Ile Asn
465                 470                 475                 480

Ala Ser Gly Val Asp Ala Lys Ala Ile Leu Ser Ala Arg Leu Ser Lys
                485                 490                 495

Ser Arg Arg Leu Glu Asn Leu Ile Ala Gln Leu Pro Gly Glu Lys Lys
            500                 505                 510

Asn Gly Leu Phe Gly Asn Leu Ile Ala Leu Ser Leu Gly Leu Thr Pro
        515                 520                 525

Asn Phe Lys Ser Asn Phe Asp Leu Ala Glu Asp Ala Lys Leu Gln Leu
    530                 535                 540

Ser Lys Asp Thr Tyr Asp Asp Asp Leu Asp Asn Leu Leu Ala Gln Ile
545                 550                 555                 560

Gly Asp Gln Tyr Ala Asp Leu Phe Leu Ala Ala Lys Asn Leu Ser Asp
                565                 570                 575

Ala Ile Leu Leu Ser Asp Ile Leu Arg Val Asn Thr Glu Ile Thr Lys
            580                 585                 590

Ala Pro Leu Ser Ala Ser Met Ile Lys Arg Tyr Asp Glu His His Gln
        595                 600                 605

Asp Leu Thr Leu Leu Lys Ala Leu Val Arg Gln Gln Leu Pro Glu Lys
    610                 615                 620

Tyr Lys Glu Ile Phe Phe Asp Gln Ser Lys Asn Gly Tyr Ala Gly Tyr
625                 630                 635                 640

Ile Asp Gly Gly Ala Ser Gln Glu Glu Phe Tyr Lys Phe Ile Lys Pro
                645                 650                 655

Ile Leu Glu Lys Met Asp Gly Thr Glu Glu Leu Leu Val Lys Leu Asn
            660                 665                 670

Arg Glu Asp Leu Leu Arg Lys Gln Arg Thr Phe Asp Asn Gly Ser Ile
        675                 680                 685

Pro His Gln Ile His Leu Gly Glu Leu His Ala Ile Leu Arg Arg Gln
    690                 695                 700

Glu Asp Phe Tyr Pro Phe Leu Lys Asp Asn Arg Glu Lys Ile Glu Lys
705                 710                 715                 720

Ile Leu Thr Phe Arg Ile Pro Tyr Tyr Val Gly Pro Leu Ala Arg Gly
                725                 730                 735

Asn Ser Arg Phe Ala Trp Met Thr Arg Lys Ser Glu Glu Thr Ile Thr
            740                 745                 750

Pro Trp Asn Phe Glu Glu Val Val Asp Lys Gly Ala Ser Ala Gln Ser
        755                 760                 765

Phe Ile Glu Arg Met Thr Asn Phe Asp Lys Asn Leu Pro Asn Glu Lys
    770                 775                 780

Val Leu Pro Lys His Ser Leu Leu Tyr Glu Tyr Phe Thr Val Tyr Asn
785                 790                 795                 800

Glu Leu Thr Lys Val Lys Tyr Val Thr Glu Gly Met Arg Lys Pro Ala
                805                 810                 815

Phe Leu Ser Gly Glu Gln Lys Lys Ala Ile Val Asp Leu Leu Phe Lys
            820                 825                 830

Thr Asn Arg Lys Val Thr Val Lys Gln Leu Lys Glu Asp Tyr Phe Lys
        835                 840                 845

Lys Ile Glu Cys Phe Asp Ser Val Glu Ile Ser Gly Val Glu Asp Arg
    850                 855                 860

Phe Asn Ala Ser Leu Gly Thr Tyr His Asp Leu Leu Lys Ile Ile Lys
865                 870                 875                 880

Asp Lys Asp Phe Leu Asp Asn Glu Glu Asn Glu Asp Ile Leu Glu Asp
                885                 890                 895

Ile Val Leu Thr Leu Thr Leu Phe Glu Asp Arg Glu Met Ile Glu Glu
            900                 905                 910

Arg Leu Lys Thr Tyr Ala His Leu Phe Asp Asp Lys Val Met Lys Gln
        915                 920                 925

Leu Lys Arg Arg Arg Tyr Thr Gly Trp Gly Arg Leu Ser Arg Lys Leu
    930                 935                 940

Ile Asn Gly Ile Arg Asp Lys Gln Ser Gly Lys Thr Ile Leu Asp Phe
945                 950                 955                 960

Leu Lys Ser Asp Gly Phe Ala Asn Arg Asn Phe Met Gln Leu Ile His
                965                 970                 975

Asp Asp Ser Leu Thr Phe Lys Glu Asp Ile Gln Lys Ala Gln Val Ser
            980                 985                 990

Gly Gln Gly Asp Ser Leu His Glu  His Ile Ala Asn Leu  Ala Gly Ser
        995                 1000                 1005

Pro Ala  Ile Lys Lys Gly Ile  Leu Gln Thr Val Lys  Val Val Asp
    1010                 1015                 1020

Glu Leu  Val Lys Val Met Gly  Arg His Lys Pro Glu  Asn Ile Val

1025                1030                1035

Ile Glu  Met Ala Arg Glu Asn  Gln Thr Thr Gln Lys  Gly Gln Lys
    1040                1045                1050

Asn Ser  Arg Glu Arg Met Lys  Arg Ile Glu Glu Gly  Ile Lys Glu
    1055                1060                1065

Leu Gly  Ser Gln Ile Leu Lys  Glu His Pro Val Glu  Asn Thr Gln
    1070                1075                1080

Leu Gln  Asn Glu Lys Leu Tyr  Leu Tyr Tyr Leu Gln  Asn Gly Arg
    1085                1090                1095

Asp Met  Tyr Val Asp Gln Glu  Leu Asp Ile Asn Arg  Leu Ser Asp
    1100                1105                1110

Tyr Asp  Val Asp His Ile Val  Pro Gln Ser Phe Leu  Lys Asp Asp
    1115                1120                1125

Ser Ile  Asp Asn Lys Val Leu  Thr Arg Ser Asp Lys  Asn Arg Gly
    1130                1135                1140

Lys Ser  Asp Asn Val Pro Ser  Glu Glu Val Val Lys  Lys Met Lys
    1145                1150                1155

Asn Tyr  Trp Arg Gln Leu Leu  Asn Ala Lys Leu Ile  Thr Gln Arg
    1160                1165                1170

Lys Phe  Asp Asn Leu Thr Lys  Ala Glu Arg Gly Gly  Leu Ser Glu
    1175                1180                1185

Leu Asp  Lys Ala Gly Phe Ile  Lys Arg Gln Leu Val  Glu Thr Arg
    1190                1195                1200

Gln Ile  Thr Lys His Val Ala  Gln Ile Leu Asp Ser  Arg Met Asn
    1205                1210                1215

Thr Lys  Tyr Asp Glu Asn Asp  Lys Leu Ile Arg Glu  Val Lys Val
    1220                1225                1230

Ile Thr  Leu Lys Ser Lys Leu  Val Ser Asp Phe Arg  Lys Asp Phe
    1235                1240                1245

Gln Phe  Tyr Lys Val Arg Glu  Ile Asn Asn Tyr His  His Ala His
    1250                1255                1260

Asp Ala Tyr Leu Asn Ala Val Val Gly Thr Ala Leu Ile Lys Lys
    1265                1270                1275

Tyr Pro Lys Leu Glu Ser Glu Phe Val Tyr Gly Asp Tyr Lys Val
    1280                1285                1290

Tyr Asp Val Arg Lys Met Ile Ala Lys Ser Glu Gln Glu Ile Gly
    1295                1300                1305

Lys Ala Thr Ala Lys Tyr Phe Phe Tyr Ser Asn Ile Met Asn Phe
    1310                1315                1320

Phe Lys Thr Glu Ile Thr Leu Ala Asn Gly Glu Ile Arg Lys Arg
    1325                1330                1335

Pro Leu Ile Glu Thr Asn Gly Glu Thr Gly Glu Ile Val Trp Asp
    1340                1345                1350

Lys Gly Arg Asp Phe Ala Thr Val Arg Lys Val Leu Ser Met Pro
    1355                1360                1365

Gln Val Asn Ile Val Lys Lys Thr Glu Val Gln Thr Gly Gly Phe
    1370                1375                1380

Ser Lys Glu Ser Ile Leu Pro Lys Arg Asn Ser Asp Lys Leu Ile
    1385                1390                1395

Ala Arg Lys Lys Asp Trp Asp Pro Lys Lys Tyr Gly Gly Phe Asp
    1400                1405                1410

Ser Pro Thr Val Ala Tyr Ser Val Leu Val Val Ala Lys Val Glu
    1415                1420                1425

Lys Gly Lys Ser Lys Lys Leu Lys Ser Val Lys Glu Leu Leu Gly
    1430                1435                1440

Ile Thr Ile Met Glu Arg Ser Ser Phe Glu Lys Asn Pro Ile Asp
    1445                1450                1455

Phe Leu Glu Ala Lys Gly Tyr Lys Glu Val Lys Lys Asp Leu Ile
    1460                1465                1470

Ile Lys Leu Pro Lys Tyr Ser Leu Phe Glu Leu Glu Asn Gly Arg
    1475                1480                1485

Lys Arg Met Leu Ala Ser Ala Gly Glu Leu Gln Lys Gly Asn Glu
    1490                1495                1500

Leu Ala Leu Pro Ser Lys Tyr Val Asn Phe Leu Tyr Leu Ala Ser
    1505                1510                1515

His Tyr Glu Lys Leu Lys Gly Ser Pro Glu Asp Asn Glu Gln Lys
    1520                1525                1530

Gln Leu Phe Val Glu Gln His Lys His Tyr Leu Asp Glu Ile Ile
    1535                1540                1545

Glu Gln Ile Ser Glu Phe Ser Lys Arg Val Ile Leu Ala Asp Ala
    1550                1555                1560

Asn Leu Asp Lys Val Leu Ser Ala Tyr Asn Lys His Arg Asp Lys
    1565                1570                1575

Pro Ile Arg Glu Gln Ala Glu Asn Ile Ile His Leu Phe Thr Leu
    1580                1585                1590

Thr Asn Leu Gly Ala Pro Ala Ala Phe Lys Tyr Phe Asp Thr Thr
    1595                1600                1605

Ile Asp Arg Lys Arg Tyr Thr Ser Thr Lys Glu Val Leu Asp Ala
    1610                1615                1620

Thr Leu Ile His Gln Ser Ile Thr Gly Leu Tyr Glu Thr Arg Ile
    1625                1630                1635

Asp Leu Ser Gln Leu Gly Gly Asp Ser Gly Gly Ser Lys Arg Thr
    1640                1645                1650

Ala Asp Gly Ser Glu Phe Glu Pro Lys Lys Lys Arg Lys Val
    1655                1660                1665

<210>  13
<211>  1827
<212>  PRT
<213>  artificial sequence

<220>
<223>   fusion protein

<400>  13

Met Lys Arg Thr Ala Asp Gly Ser Glu Phe Glu Ser Pro Lys Lys Lys
1               5                   10                  15

Arg Lys Val Ser Ser Glu Thr Gly Pro Val Ala Val Asp Pro Thr Leu
            20                  25                  30

Arg Arg Arg Ile Glu Pro His Glu Phe Glu Val Phe Phe Asp Pro Arg
        35                  40                  45

Glu Leu Arg Lys Glu Thr Cys Leu Leu Tyr Glu Ile Asn Trp Gly Gly
    50                  55                  60

Arg His Ser Ile Trp Arg His Thr Ser Gln Asn Thr Asn Lys His Val
65                  70                  75                  80

Glu Val Asn Phe Ile Glu Lys Phe Thr Thr Glu Arg Tyr Phe Cys Pro
                85                  90                  95

Asn Thr Arg Cys Ser Ile Thr Trp Phe Leu Ser Trp Ser Pro Cys Gly
            100                 105                 110

Glu Cys Ser Arg Ala Ile Thr Glu Phe Leu Ser Arg Tyr Pro His Val
        115                 120                 125

Thr Leu Phe Ile Tyr Ile Ala Arg Leu Tyr His His Ala Asp Pro Arg
    130                 135                 140

Asn Arg Gln Gly Leu Arg Asp Leu Ile Ser Ser Gly Val Thr Ile Gln
145                 150                 155                 160

Ile Met Thr Glu Gln Glu Ser Gly Tyr Cys Trp Arg Asn Phe Val Asn
                165                 170                 175

Tyr Ser Pro Ser Asn Glu Ala His Trp Pro Arg Tyr Pro His Leu Trp
            180                 185                 190

Val Arg Leu Tyr Val Leu Glu Leu Tyr Cys Ile Ile Leu Gly Leu Pro
        195                 200                 205

Pro Cys Leu Asn Ile Leu Arg Arg Lys Gln Pro Gln Leu Thr Phe Phe
    210                 215                 220

Thr Ile Ala Leu Gln Ser Cys His Tyr Gln Arg Leu Pro Pro His Ile
225                 230                 235                 240

Leu Trp Ala Thr Gly Leu Lys Ser Gly Gly Ser Ser Gly Gly Ser Ser
                245                 250                 255

Gly Ser Glu Thr Pro Gly Thr Ser Glu Ser Ala Thr Pro Glu Ser Ser
            260                 265                 270

Gly Gly Ser Ser Gly Gly Ser Asp Lys Lys Tyr Ser Ile Gly Leu Ala
        275                 280                 285

Ile Gly Thr Asn Ser Val Gly Trp Ala Val Ile Thr Asp Glu Tyr Lys
    290                 295                 300

Val Pro Ser Lys Lys Phe Lys Val Leu Gly Asn Thr Asp Arg His Ser
305                 310                 315                 320

Ile Lys Lys Asn Leu Ile Gly Ala Leu Leu Phe Asp Ser Gly Glu Thr
                325                 330                 335

Ala Glu Ala Thr Arg Leu Lys Arg Thr Ala Arg Arg Arg Tyr Thr Arg
            340                 345                 350

Arg Lys Asn Arg Ile Cys Tyr Leu Gln Glu Ile Phe Ser Asn Glu Met
        355                 360                 365

Ala Lys Val Asp Asp Ser Phe Phe His Arg Leu Glu Glu Ser Phe Leu
    370                 375                 380

Val Glu Glu Asp Lys Lys His Glu Arg His Pro Ile Phe Gly Asn Ile
385                 390                 395                 400

Val Asp Glu Val Ala Tyr His Glu Lys Tyr Pro Thr Ile Tyr His Leu
                405                 410                 415

Arg Lys Lys Leu Val Asp Ser Thr Asp Lys Ala Asp Leu Arg Leu Ile
            420                 425                 430

Tyr Leu Ala Leu Ala His Met Ile Lys Phe Arg Gly His Phe Leu Ile
        435                 440                 445

Glu Gly Asp Leu Asn Pro Asp Asn Ser Asp Val Asp Lys Leu Phe Ile
    450                 455                 460

Gln Leu Val Gln Thr Tyr Asn Gln Leu Phe Glu Glu Asn Pro Ile Asn
465                 470                 475                 480

Ala Ser Gly Val Asp Ala Lys Ala Ile Leu Ser Ala Arg Leu Ser Lys
                485                 490                 495

Ser Arg Arg Leu Glu Asn Leu Ile Ala Gln Leu Pro Gly Glu Lys Lys
            500                 505                 510

Asn Gly Leu Phe Gly Asn Leu Ile Ala Leu Ser Leu Gly Leu Thr Pro
        515                 520                 525

Asn Phe Lys Ser Asn Phe Asp Leu Ala Glu Asp Ala Lys Leu Gln Leu
    530                 535                 540

Ser Lys Asp Thr Tyr Asp Asp Asp Leu Asp Asn Leu Leu Ala Gln Ile
545                 550                 555                 560

Gly Asp Gln Tyr Ala Asp Leu Phe Leu Ala Ala Lys Asn Leu Ser Asp
                565                 570                 575

Ala Ile Leu Leu Ser Asp Ile Leu Arg Val Asn Thr Glu Ile Thr Lys
            580                 585                 590

Ala Pro Leu Ser Ala Ser Met Ile Lys Arg Tyr Asp Glu His His Gln
        595                 600                 605

Asp Leu Thr Leu Leu Lys Ala Leu Val Arg Gln Gln Leu Pro Glu Lys
    610                 615                 620

Tyr Lys Glu Ile Phe Phe Asp Gln Ser Lys Asn Gly Tyr Ala Gly Tyr
625                 630                 635                 640

Ile Asp Gly Gly Ala Ser Gln Glu Glu Phe Tyr Lys Phe Ile Lys Pro
                645                 650                 655

Ile Leu Glu Lys Met Asp Gly Thr Glu Glu Leu Leu Val Lys Leu Asn
            660                 665                 670

Arg Glu Asp Leu Leu Arg Lys Gln Arg Thr Phe Asp Asn Gly Ser Ile
        675                 680                 685

Pro His Gln Ile His Leu Gly Glu Leu His Ala Ile Leu Arg Arg Gln
    690                 695                 700

Glu Asp Phe Tyr Pro Phe Leu Lys Asp Asn Arg Glu Lys Ile Glu Lys
705                 710                 715                 720

Ile Leu Thr Phe Arg Ile Pro Tyr Tyr Val Gly Pro Leu Ala Arg Gly
                725                 730                 735

Asn Ser Arg Phe Ala Trp Met Thr Arg Lys Ser Glu Glu Thr Ile Thr
            740                 745                 750

Pro Trp Asn Phe Glu Glu Val Val Asp Lys Gly Ala Ser Ala Gln Ser
        755                 760                 765

Phe Ile Glu Arg Met Thr Asn Phe Asp Lys Asn Leu Pro Asn Glu Lys
    770                 775                 780

Val Leu Pro Lys His Ser Leu Leu Tyr Glu Tyr Phe Thr Val Tyr Asn

785                 790                 795                 800

Glu Leu Thr Lys Val Lys Tyr Val Thr Glu Gly Met Arg Lys Pro Ala
                805                 810                 815

Phe Leu Ser Gly Glu Gln Lys Lys Ala Ile Val Asp Leu Leu Phe Lys
            820                 825                 830

Thr Asn Arg Lys Val Thr Val Lys Gln Leu Lys Glu Asp Tyr Phe Lys
        835                 840                 845

Lys Ile Glu Cys Phe Asp Ser Val Glu Ile Ser Gly Val Glu Asp Arg
    850                 855                 860

Phe Asn Ala Ser Leu Gly Thr Tyr His Asp Leu Leu Lys Ile Ile Lys
865                 870                 875                 880

Asp Lys Asp Phe Leu Asp Asn Glu Glu Asn Glu Asp Ile Leu Glu Asp
                885                 890                 895

Ile Val Leu Thr Leu Thr Leu Phe Glu Asp Arg Glu Met Ile Glu Glu
            900                 905                 910

Arg Leu Lys Thr Tyr Ala His Leu Phe Asp Asp Lys Val Met Lys Gln
        915                 920                 925

Leu Lys Arg Arg Arg Tyr Thr Gly Trp Gly Arg Leu Ser Arg Lys Leu
    930                 935                 940

Ile Asn Gly Ile Arg Asp Lys Gln Ser Gly Lys Thr Ile Leu Asp Phe
945                 950                 955                 960

Leu Lys Ser Asp Gly Phe Ala Asn Arg Asn Phe Met Gln Leu Ile His
                965                 970                 975

Asp Asp Ser Leu Thr Phe Lys Glu Asp Ile Gln Lys Ala Gln Val Ser
            980                 985                 990

Gly Gln Gly Asp Ser Leu His Glu  His Ile Ala Asn Leu  Ala Gly Ser
        995                 1000                 1005

Pro Ala  Ile Lys Lys Gly Ile  Leu Gln Thr Val Lys  Val Val Asp
    1010                 1015                 1020

Glu Leu  Val Lys Val Met Gly  Arg His Lys Pro Glu  Asn Ile Val
    1025                 1030                 1035

Ile Glu Met Ala Arg Glu Asn Gln Thr Thr Gln Lys Gly Gln Lys
    1040                1045                1050

Asn Ser Arg Glu Arg Met Lys Arg Ile Glu Glu Gly Ile Lys Glu
    1055                1060                1065

Leu Gly Ser Gln Ile Leu Lys Glu His Pro Val Glu Asn Thr Gln
    1070                1075                1080

Leu Gln Asn Glu Lys Leu Tyr Leu Tyr Tyr Leu Gln Asn Gly Arg
    1085                1090                1095

Asp Met Tyr Val Asp Gln Glu Leu Asp Ile Asn Arg Leu Ser Asp
    1100                1105                1110

Tyr Asp Val Asp His Ile Val Pro Gln Ser Phe Leu Lys Asp Asp
    1115                1120                1125

Ser Ile Asp Asn Lys Val Leu Thr Arg Ser Asp Lys Asn Arg Gly
    1130                1135                1140

Lys Ser Asp Asn Val Pro Ser Glu Glu Val Val Lys Lys Met Lys
    1145                1150                1155

Asn Tyr Trp Arg Gln Leu Leu Asn Ala Lys Leu Ile Thr Gln Arg
    1160                1165                1170

Lys Phe Asp Asn Leu Thr Lys Ala Glu Arg Gly Gly Leu Ser Glu
    1175                1180                1185

Leu Asp Lys Ala Gly Phe Ile Lys Arg Gln Leu Val Glu Thr Arg
    1190                1195                1200

Gln Ile Thr Lys His Val Ala Gln Ile Leu Asp Ser Arg Met Asn
    1205                1210                1215

Thr Lys Tyr Asp Glu Asn Asp Lys Leu Ile Arg Glu Val Lys Val
    1220                1225                1230

Ile Thr Leu Lys Ser Lys Leu Val Ser Asp Phe Arg Lys Asp Phe
    1235                1240                1245

Gln Phe Tyr Lys Val Arg Glu Ile Asn Asn Tyr His His Ala His
    1250                1255                1260

Asp Ala Tyr Leu Asn Ala Val Val Gly Thr Ala Leu Ile Lys Lys
    1265                1270                1275

Tyr Pro Lys Leu Glu Ser Glu Phe Val Tyr Gly Asp Tyr Lys Val
    1280                1285                1290

Tyr Asp Val Arg Lys Met Ile Ala Lys Ser Glu Gln Glu Ile Gly
    1295                1300                1305

Lys Ala Thr Ala Lys Tyr Phe Phe Tyr Ser Asn Ile Met Asn Phe
    1310                1315                1320

Phe Lys Thr Glu Ile Thr Leu Ala Asn Gly Glu Ile Arg Lys Arg
    1325                1330                1335

Pro Leu Ile Glu Thr Asn Gly Glu Thr Gly Glu Ile Val Trp Asp
    1340                1345                1350

Lys Gly Arg Asp Phe Ala Thr Val Arg Lys Val Leu Ser Met Pro
    1355                1360                1365

Gln Val Asn Ile Val Lys Lys Thr Glu Val Gln Thr Gly Gly Phe
    1370                1375                1380

Ser Lys Glu Ser Ile Leu Pro Lys Arg Asn Ser Asp Lys Leu Ile
    1385                1390                1395

Ala Arg Lys Lys Asp Trp Asp Pro Lys Lys Tyr Gly Gly Phe Asp
    1400                1405                1410

Ser Pro Thr Val Ala Tyr Ser Val Leu Val Val Ala Lys Val Glu
    1415                1420                1425

Lys Gly Lys Ser Lys Lys Leu Lys Ser Val Lys Glu Leu Leu Gly
    1430                1435                1440

Ile Thr Ile Met Glu Arg Ser Ser Phe Glu Lys Asn Pro Ile Asp
    1445                1450                1455

Phe Leu Glu Ala Lys Gly Tyr Lys Glu Val Lys Lys Asp Leu Ile
    1460                1465                1470

Ile Lys Leu Pro Lys Tyr Ser Leu Phe Glu Leu Glu Asn Gly Arg
    1475                1480                1485

Lys Arg Met Leu Ala Ser Ala Gly Glu Leu Gln Lys Gly Asn Glu
    1490                1495                1500

Leu Ala Leu Pro Ser Lys Tyr Val Asn Phe Leu Tyr Leu Ala Ser
    1505                1510                1515

His Tyr Glu Lys Leu Lys Gly Ser Pro Glu Asp Asn Glu Gln Lys
    1520                1525                1530

Gln Leu Phe Val Glu Gln His Lys His Tyr Leu Asp Glu Ile Ile
    1535                1540                1545

Glu Gln Ile Ser Glu Phe Ser Lys Arg Val Ile Leu Ala Asp Ala
    1550                1555                1560

Asn Leu Asp Lys Val Leu Ser Ala Tyr Asn Lys His Arg Asp Lys
    1565                1570                1575

Pro Ile Arg Glu Gln Ala Glu Asn Ile Ile His Leu Phe Thr Leu
    1580                1585                1590

Thr Asn Leu Gly Ala Pro Ala Ala Phe Lys Tyr Phe Asp Thr Thr
    1595                1600                1605

Ile Asp Arg Lys Arg Tyr Thr Ser Thr Lys Glu Val Leu Asp Ala
    1610                1615                1620

Thr Leu Ile His Gln Ser Ile Thr Gly Leu Tyr Glu Thr Arg Ile
    1625                1630                1635

Asp Leu Ser Gln Leu Gly Gly Asp Ser Gly Gly Ser Lys Arg Thr
    1640                1645                1650

Ala Asp Gly Ser Glu Phe Glu Pro Lys Lys Lys Arg Lys Val Ser
    1655                1660                1665

Gly Gly Ser Ser Gly Gly Ser Ser Gly Ser Glu Thr Pro Gly Thr
    1670                1675                1680

Ser Glu Ser Ala Thr Pro Glu Ser Ser Gly Ser Glu Thr Pro Gly
    1685                1690                1695

Thr Ser Glu Ser Ala Thr Pro Glu Ser Ser Gly Gly Ser Ser Gly
    1700                1705                1710

Gly Ser Met Ser Asp Lys Pro Lys Arg Pro Leu Ser Ala Tyr Met
    1715                1720                1725

Leu Trp Leu Asn Ser Ala Arg Glu Ser Ile Lys Arg Glu Asn Pro
    1730                1735                1740

Gly Ile Lys Val Thr Glu Val Ala Lys Arg Gly Gly Glu Leu Trp

1745                    1750                    1755

Arg Ala  Met Lys Asp Lys Ser  Glu Trp Glu Ala Lys  Ala Ala Lys
    1760                    1765                    1770

Ala Lys  Asp Asp Tyr Asp Arg  Ala Val Lys Glu Phe  Glu Ala Asn
    1775                    1780                    1785

Gly Gly  Ser Ser Ala Ala Asn  Gly Gly Gly Ala Lys  Lys Arg Ala
    1790                    1795                    1800

Lys Pro  Ala Lys Lys Val Ala  Lys Lys Ser Lys Lys  Glu Glu Ser
    1805                    1810                    1815

Asp Glu  Asp Asp Asp Asp Glu  Ser Glu
    1820                    1825

<210>  14
<211>  1057
<212>  PRT
<213>  Staphylococcus aureus

<400>  14

Met Glu Ala Ser Met Lys Arg Asn Tyr Ile Leu Gly Leu Asp Ile Gly
1               5                   10                  15

Ile Thr Ser Val Gly Tyr Gly Ile Ile Asp Tyr Glu Thr Arg Asp Val
            20                  25                  30

Ile Asp Ala Gly Val Arg Leu Phe Lys Glu Ala Asn Val Glu Asn Asn
        35                  40                  45

Glu Gly Arg Arg Ser Lys Arg Gly Ala Arg Arg Leu Lys Arg Arg Arg
    50                  55                  60

Arg His Arg Ile Gln Arg Val Lys Lys Leu Leu Phe Asp Tyr Asn Leu
65                  70                  75                  80

Leu Thr Asp His Ser Glu Leu Ser Gly Ile Asn Pro Tyr Glu Ala Arg
                85                  90                  95

Val Lys Gly Leu Ser Gln Lys Leu Ser Glu Glu Glu Phe Ser Ala Ala
            100                 105                 110

Leu Leu His Leu Ala Lys Arg Arg Gly Val His Asn Val Asn Glu Val
        115                 120                 125

Glu Glu Asp Thr Gly Asn Glu Leu Ser Thr Lys Glu Gln Ile Ser Arg

130                 135                 140

Asn Ser Lys Ala Leu Glu Glu Lys Tyr Val Ala Glu Leu Gln Leu Glu
145                 150                 155                 160

Arg Leu Lys Lys Asp Gly Glu Val Arg Gly Ser Ile Asn Arg Phe Lys
                165                 170                 175

Thr Ser Asp Tyr Val Lys Glu Ala Lys Gln Leu Leu Lys Val Gln Lys
            180                 185                 190

Ala Tyr His Gln Leu Asp Gln Ser Phe Ile Asp Thr Tyr Ile Asp Leu
        195                 200                 205

Leu Glu Thr Arg Arg Thr Tyr Tyr Glu Gly Pro Gly Glu Gly Ser Pro
    210                 215                 220

Phe Gly Trp Lys Asp Ile Lys Glu Trp Tyr Glu Met Leu Met Gly His
225                 230                 235                 240

Cys Thr Tyr Phe Pro Glu Glu Leu Arg Ser Val Lys Tyr Ala Tyr Asn
                245                 250                 255

Ala Asp Leu Tyr Asn Ala Leu Asn Asp Leu Asn Asn Leu Val Ile Thr
            260                 265                 270

Arg Asp Glu Asn Glu Lys Leu Glu Tyr Tyr Glu Lys Phe Gln Ile Ile
        275                 280                 285

Glu Asn Val Phe Lys Gln Lys Lys Lys Pro Thr Leu Lys Gln Ile Ala
    290                 295                 300

Lys Glu Ile Leu Val Asn Glu Glu Asp Ile Lys Gly Tyr Arg Val Thr
305                 310                 315                 320

Ser Thr Gly Lys Pro Glu Phe Thr Asn Leu Lys Val Tyr His Asp Ile
                325                 330                 335

Lys Asp Ile Thr Ala Arg Lys Glu Ile Ile Glu Asn Ala Glu Leu Leu
            340                 345                 350

Asp Gln Ile Ala Lys Ile Leu Thr Ile Tyr Gln Ser Ser Glu Asp Ile
        355                 360                 365

Gln Glu Glu Leu Thr Asn Leu Asn Ser Glu Leu Thr Gln Glu Glu Ile
    370                 375                 380

Glu Gln Ile Ser Asn Leu Lys Gly Tyr Thr Gly Thr His Asn Leu Ser
385                 390                 395                 400

Leu Lys Ala Ile Asn Leu Ile Leu Asp Glu Leu Trp His Thr Asn Asp
                405                 410                 415

Asn Gln Ile Ala Ile Phe Asn Arg Leu Lys Leu Val Pro Lys Lys Val
            420                 425                 430

Asp Leu Ser Gln Gln Lys Glu Ile Pro Thr Thr Leu Val Asp Asp Phe
        435                 440                 445

Ile Leu Ser Pro Val Val Lys Arg Ser Phe Ile Gln Ser Ile Lys Val
    450                 455                 460

Ile Asn Ala Ile Ile Lys Lys Tyr Gly Leu Pro Asn Asp Ile Ile Ile
465                 470                 475                 480

Glu Leu Ala Arg Glu Lys Asn Ser Lys Asp Ala Gln Lys Met Ile Asn
                485                 490                 495

Glu Met Gln Lys Arg Asn Arg Gln Thr Asn Glu Arg Ile Glu Glu Ile
            500                 505                 510

Ile Arg Thr Thr Gly Lys Glu Asn Ala Lys Tyr Leu Ile Glu Lys Ile
        515                 520                 525

Lys Leu His Asp Met Gln Glu Gly Lys Cys Leu Tyr Ser Leu Glu Ala
    530                 535                 540

Ile Pro Leu Glu Asp Leu Leu Asn Asn Pro Phe Asn Tyr Glu Val Asp
545                 550                 555                 560

His Ile Ile Pro Arg Ser Val Ser Phe Asp Asn Ser Phe Asn Asn Lys
                565                 570                 575

Val Leu Val Lys Gln Glu Glu Asn Ser Lys Lys Gly Asn Arg Thr Pro
            580                 585                 590

Phe Gln Tyr Leu Ser Ser Ser Asp Ser Lys Ile Ser Tyr Glu Thr Phe
        595                 600                 605

Lys Lys His Ile Leu Asn Leu Ala Lys Gly Lys Gly Arg Ile Ser Lys
    610                 615                 620

Thr Lys Lys Glu Tyr Leu Leu Glu Glu Arg Asp Ile Asn Arg Phe Ser
625                 630                 635                 640

Val Gln Lys Asp Phe Ile Asn Arg Asn Leu Val Asp Thr Arg Tyr Ala
                645                 650                 655

Thr Arg Gly Leu Met Asn Leu Leu Arg Ser Tyr Phe Arg Val Asn Asn
            660                 665                 670

Leu Asp Val Lys Val Lys Ser Ile Asn Gly Gly Phe Thr Ser Phe Leu
        675                 680                 685

Arg Arg Lys Trp Lys Phe Lys Lys Glu Arg Asn Lys Gly Tyr Lys His
    690                 695                 700

His Ala Glu Asp Ala Leu Ile Ile Ala Asn Ala Asp Phe Ile Phe Lys
705                 710                 715                 720

Glu Trp Lys Lys Leu Asp Lys Ala Lys Lys Val Met Glu Asn Gln Met
                725                 730                 735

Phe Glu Glu Lys Gln Ala Glu Ser Met Pro Glu Ile Glu Thr Glu Gln
            740                 745                 750

Glu Tyr Lys Glu Ile Phe Ile Thr Pro His Gln Ile Lys His Ile Lys
        755                 760                 765

Asp Phe Lys Asp Tyr Lys Tyr Ser His Arg Val Asp Lys Lys Pro Asn
    770                 775                 780

Arg Glu Leu Ile Asn Asp Thr Leu Tyr Ser Thr Arg Lys Asp Asp Lys
785                 790                 795                 800

Gly Asn Thr Leu Ile Val Asn Asn Leu Asn Gly Leu Tyr Asp Lys Asp
                805                 810                 815

Asn Asp Lys Leu Lys Lys Leu Ile Asn Lys Ser Pro Glu Lys Leu Leu
            820                 825                 830

Met Tyr His His Asp Pro Gln Thr Tyr Gln Lys Leu Lys Leu Ile Met
        835                 840                 845

Glu Gln Tyr Gly Asp Glu Lys Asn Pro Leu Tyr Lys Tyr Tyr Glu Glu
    850                 855                 860

Thr Gly Asn Tyr Leu Thr Lys Tyr Ser Lys Lys Asp Asn Gly Pro Val
865                 870                 875                 880

Ile Lys Lys Ile Lys Tyr Tyr Gly Asn Lys Leu Asn Ala His Leu Asp
                885                 890                 895

Ile Thr Asp Asp Tyr Pro Asn Ser Arg Asn Lys Val Val Lys Leu Ser
                900                 905                 910

Leu Lys Pro Tyr Arg Phe Asp Val Tyr Leu Asp Asn Gly Val Tyr Lys
        915                 920                 925

Phe Val Thr Val Lys Asn Leu Asp Val Ile Lys Lys Glu Asn Tyr Tyr
    930                 935                 940

Glu Val Asn Ser Lys Cys Tyr Glu Glu Ala Lys Lys Leu Lys Lys Ile
945                 950                 955                 960

Ser Asn Gln Ala Glu Phe Ile Ala Ser Phe Tyr Asn Asn Asp Leu Ile
                965                 970                 975

Lys Ile Asn Gly Glu Leu Tyr Arg Val Ile Gly Val Asn Asn Asp Leu
                980                 985                 990

Leu Asn Arg Ile Glu Val Asn Met  Ile Asp Ile Thr Tyr  Arg Glu Tyr
        995                 1000                 1005

Leu Glu  Asn Met Asn Asp Lys  Arg Pro Pro Arg Ile  Ile Lys Thr
    1010                 1015                 1020

Ile Ala  Ser Lys Thr Gln Ser  Ile Lys Lys Tyr Ser  Thr Asp Ile
    1025                 1030                 1035

Leu Gly  Asn Leu Tyr Glu Val  Lys Ser Lys Lys His  Pro Gln Ile
    1040                 1045                 1050

Ile Lys  Lys Gly
    1055

<210>  15
<211>  1307
<212>  PRT
<213>  Acidaminococcus sp

<400>  15

Met Thr Gln Phe Glu Gly Phe Thr Asn Leu Tyr Gln Val Ser Lys Thr
1               5                   10                  15

Leu Arg Phe Glu Leu Ile Pro Gln Gly Lys Thr Leu Lys His Ile Gln
            20                  25                  30

Glu Gln Gly Phe Ile Glu Glu Asp Lys Ala Arg Asn Asp His Tyr Lys
        35                  40                  45

Glu Leu Lys Pro Ile Ile Asp Arg Ile Tyr Lys Thr Tyr Ala Asp Gln
    50                  55                  60

Cys Leu Gln Leu Val Gln Leu Asp Trp Glu Asn Leu Ser Ala Ala Ile
65                  70                  75                  80

Asp Ser Tyr Arg Lys Glu Lys Thr Glu Glu Thr Arg Asn Ala Leu Ile
                85                  90                  95

Glu Glu Gln Ala Thr Tyr Arg Asn Ala Ile His Asp Tyr Phe Ile Gly
            100                 105                 110

Arg Thr Asp Asn Leu Thr Asp Ala Ile Asn Lys Arg His Ala Glu Ile
        115                 120                 125

Tyr Lys Gly Leu Phe Lys Ala Glu Leu Phe Asn Gly Lys Val Leu Lys
    130                 135                 140

Gln Leu Gly Thr Val Thr Thr Thr Glu His Glu Asn Ala Leu Leu Arg
145                 150                 155                 160

Ser Phe Asp Lys Phe Thr Thr Tyr Phe Ser Gly Phe Tyr Glu Asn Arg
                165                 170                 175

Lys Asn Val Phe Ser Ala Glu Asp Ile Ser Thr Ala Ile Pro His Arg
            180                 185                 190

Ile Val Gln Asp Asn Phe Pro Lys Phe Lys Glu Asn Cys His Ile Phe
        195                 200                 205

Thr Arg Leu Ile Thr Ala Val Pro Ser Leu Arg Glu His Phe Glu Asn
    210                 215                 220

Val Lys Lys Ala Ile Gly Ile Phe Val Ser Thr Ser Ile Glu Glu Val
225                 230                 235                 240

Phe Ser Phe Pro Phe Tyr Asn Gln Leu Leu Thr Gln Thr Gln Ile Asp
                245                 250                 255

Leu Tyr Asn Gln Leu Leu Gly Gly Ile Ser Arg Glu Ala Gly Thr Glu
            260                 265                 270

Lys Ile Lys Gly Leu Asn Glu Val Leu Asn Leu Ala Ile Gln Lys Asn
        275                 280                 285

Asp Glu Thr Ala His Ile Ile Ala Ser Leu Pro His Arg Phe Ile Pro

290                 295                 300

Leu Phe Lys Gln Ile Leu Ser Asp Arg Asn Thr Leu Ser Phe Ile Leu
305                 310                 315                 320

Glu Glu Phe Lys Ser Asp Glu Glu Val Ile Gln Ser Phe Cys Lys Tyr
                325                 330                 335

Lys Thr Leu Leu Arg Asn Glu Asn Val Leu Glu Thr Ala Glu Ala Leu
            340                 345                 350

Phe Asn Glu Leu Asn Ser Ile Asp Leu Thr His Ile Phe Ile Ser His
        355                 360                 365

Lys Lys Leu Glu Thr Ile Ser Ser Ala Leu Cys Asp His Trp Asp Thr
    370                 375                 380

Leu Arg Asn Ala Leu Tyr Glu Arg Arg Ile Ser Glu Leu Thr Gly Lys
385                 390                 395                 400

Ile Thr Lys Ser Ala Lys Glu Lys Val Gln Arg Ser Leu Lys His Glu
                405                 410                 415

Asp Ile Asn Leu Gln Glu Ile Ile Ser Ala Ala Gly Lys Glu Leu Ser
            420                 425                 430

Glu Ala Phe Lys Gln Lys Thr Ser Glu Ile Leu Ser His Ala His Ala
        435                 440                 445

Ala Leu Asp Gln Pro Leu Pro Thr Thr Leu Lys Lys Gln Glu Glu Lys
    450                 455                 460

Glu Ile Leu Lys Ser Gln Leu Asp Ser Leu Leu Gly Leu Tyr His Leu
465                 470                 475                 480

Leu Asp Trp Phe Ala Val Asp Glu Ser Asn Glu Val Asp Pro Glu Phe
                485                 490                 495

Ser Ala Arg Leu Thr Gly Ile Lys Leu Glu Met Glu Pro Ser Leu Ser
            500                 505                 510

Phe Tyr Asn Lys Ala Arg Asn Tyr Ala Thr Lys Lys Pro Tyr Ser Val
        515                 520                 525

Glu Lys Phe Lys Leu Asn Phe Gln Met Pro Thr Leu Ala Ser Gly Trp
    530                 535                 540

Asp Val Asn Lys Glu Lys Asn Asn Gly Ala Ile Leu Phe Val Lys Asn
545                 550                 555                 560

Gly Leu Tyr Tyr Leu Gly Ile Met Pro Lys Gln Lys Gly Arg Tyr Lys
                565                 570                 575

Ala Leu Ser Phe Glu Pro Thr Glu Lys Thr Ser Glu Gly Phe Asp Lys
            580                 585                 590

Met Tyr Tyr Asp Tyr Phe Pro Asp Ala Ala Lys Met Ile Pro Lys Cys
        595                 600                 605

Ser Thr Gln Leu Lys Ala Val Thr Ala His Phe Gln Thr His Thr Thr
    610                 615                 620

Pro Ile Leu Leu Ser Asn Asn Phe Ile Glu Pro Leu Glu Ile Thr Lys
625                 630                 635                 640

Glu Ile Tyr Asp Leu Asn Asn Pro Glu Lys Glu Pro Lys Lys Phe Gln
                645                 650                 655

Thr Ala Tyr Ala Lys Lys Thr Gly Asp Gln Lys Gly Tyr Arg Glu Ala
            660                 665                 670

Leu Cys Lys Trp Ile Asp Phe Thr Arg Asp Phe Leu Ser Lys Tyr Thr
        675                 680                 685

Lys Thr Thr Ser Ile Asp Leu Ser Ser Leu Arg Pro Ser Ser Gln Tyr
    690                 695                 700

Lys Asp Leu Gly Glu Tyr Tyr Ala Glu Leu Asn Pro Leu Leu Tyr His
705                 710                 715                 720

Ile Ser Phe Gln Arg Ile Ala Glu Lys Glu Ile Met Asp Ala Val Glu
                725                 730                 735

Thr Gly Lys Leu Tyr Leu Phe Gln Ile Tyr Asn Lys Asp Phe Ala Lys
            740                 745                 750

Gly His His Gly Lys Pro Asn Leu His Thr Leu Tyr Trp Thr Gly Leu
        755                 760                 765

Phe Ser Pro Glu Asn Leu Ala Lys Thr Ser Ile Lys Leu Asn Gly Gln
    770                 775                 780

Ala Glu Leu Phe Tyr Arg Pro Lys Ser Arg Met Lys Arg Met Ala His
785                 790                 795                 800

Arg Leu Gly Glu Lys Met Leu Asn Lys Lys Leu Lys Asp Gln Lys Thr
                805                 810                 815

Pro Ile Pro Asp Thr Leu Tyr Gln Glu Leu Tyr Asp Tyr Val Asn His
            820                 825                 830

Arg Leu Ser His Asp Leu Ser Asp Glu Ala Arg Ala Leu Leu Pro Asn
        835                 840                 845

Val Ile Thr Lys Glu Val Ser His Glu Ile Ile Lys Asp Arg Arg Phe
    850                 855                 860

Thr Ser Asp Lys Phe Phe Phe His Val Pro Ile Thr Leu Asn Tyr Gln
865                 870                 875                 880

Ala Ala Asn Ser Pro Ser Lys Phe Asn Gln Arg Val Asn Ala Tyr Leu
                885                 890                 895

Lys Glu His Pro Glu Thr Pro Ile Ile Gly Ile Asp Arg Gly Glu Arg
            900                 905                 910

Asn Leu Ile Tyr Ile Thr Val Ile Asp Ser Thr Gly Lys Ile Leu Glu
        915                 920                 925

Gln Arg Ser Leu Asn Thr Ile Gln Gln Phe Asp Tyr Gln Lys Lys Leu
    930                 935                 940

Asp Asn Arg Glu Lys Glu Arg Val Ala Ala Arg Gln Ala Trp Ser Val
945                 950                 955                 960

Val Gly Thr Ile Lys Asp Leu Lys Gln Gly Tyr Leu Ser Gln Val Ile
                965                 970                 975

His Glu Ile Val Asp Leu Met Ile His Tyr Gln Ala Val Val Val Leu
            980                 985                 990

Glu Asn Leu Asn Phe Gly Phe Lys  Ser Lys Arg Thr Gly  Ile Ala Glu
        995                 1000                 1005

Lys Ala  Val Tyr Gln Gln Phe  Glu Lys Met Leu Ile  Asp Lys Leu
    1010                 1015                 1020

Asn Cys  Leu Val Leu Lys Asp  Tyr Pro Ala Glu Lys  Val Gly Gly
    1025                 1030                 1035

Val Leu  Asn Pro Tyr Gln Leu  Thr Asp Gln Phe Thr  Ser Phe Ala
    1040                 1045                 1050

Lys Met Gly Thr Gln Ser Gly Phe Leu Phe Tyr Val Pro Ala Pro
    1055                1060                1065

Tyr Thr Ser Lys Ile Asp Pro Leu Thr Gly Phe Val Asp Pro Phe
    1070                1075                1080

Val Trp Lys Thr Ile Lys Asn His Glu Ser Arg Lys His Phe Leu
    1085                1090                1095

Glu Gly Phe Asp Phe Leu His Tyr Asp Val Lys Thr Gly Asp Phe
    1100                1105                1110

Ile Leu His Phe Lys Met Asn Arg Asn Leu Ser Phe Gln Arg Gly
    1115                1120                1125

Leu Pro Gly Phe Met Pro Ala Trp Asp Ile Val Phe Glu Lys Asn
    1130                1135                1140

Glu Thr Gln Phe Asp Ala Lys Gly Thr Pro Phe Ile Ala Gly Lys
    1145                1150                1155

Arg Ile Val Pro Val Ile Glu Asn His Arg Phe Thr Gly Arg Tyr
    1160                1165                1170

Arg Asp Leu Tyr Pro Ala Asn Glu Leu Ile Ala Leu Leu Glu Glu
    1175                1180                1185

Lys Gly Ile Val Phe Arg Asp Gly Ser Asn Ile Leu Pro Lys Leu
    1190                1195                1200

Leu Glu Asn Asp Asp Ser His Ala Ile Asp Thr Met Val Ala Leu
    1205                1210                1215

Ile Arg Ser Val Leu Gln Met Arg Asn Ser Asn Ala Ala Thr Gly
    1220                1225                1230

Glu Asp Tyr Ile Asn Ser Pro Val Arg Asp Leu Asn Gly Val Cys
    1235                1240                1245

Phe Asp Ser Arg Phe Gln Asn Pro Glu Trp Pro Met Asp Ala Asp
    1250                1255                1260

Ala Asn Gly Ala Tyr His Ile Ala Leu Lys Gly Gln Leu Leu Leu
    1265                1270                1275

Asn His Leu Lys Glu Ser Lys Asp Leu Lys Leu Gln Asn Gly Ile

1280                1285                1290

Ser Asn Gln Asp Trp Leu Ala Tyr Ile Gln Glu Leu Arg Asn
    1295                1300                1305